(19) Europäisches Patentamt
European Patent Office
Office européen des brevets

(11) Publication number: 0 289 262 A2

(12) # EUROPEAN PATENT APPLICATION

(21) Application number: 88303741.8

(22) Date of filing: 26.04.88

(51) Int. Cl.[4]: C 07 C 93/00
C 07 C 153/00, C 07 C 93/20,
C 07 C 149/40,
C 07 C 101/50,
C 07 C 101/44,
C 07 D 209/26,
C 07 D 207/337,
C 07 D 263/56,
C 07 D 405/04, C 07 D 487/04

(30) Priority: 27.04.87 US 43072

(43) Date of publication of application:
02.11.88 Bulletin 88/44

(84) Designated Contracting States:
AT BE CH DE ES FR GB GR IT LI LU NL SE

(71) Applicant: SYNTEX PHARMACEUTICALS INTERNATIONAL LIMITED
Corner House Church Street
Hamilton 5 (BM)

(72) Inventor: Venuti, Michael C.
11 Hillview Ct.
San Francisco California 94124 (US)

Young, John M.
300 Summit Dr.
Redwood City California 94062 (US)

(74) Representative: Armitage, Ian Michael et al
MEWBURN ELLIS & CO. 2/3 Cursitor Street
London EC4A 1BQ (GB)

Claims for the following Contracting States: ES + GR.

(54) **Omega-quaternary ammonium alkyl esters and thioesters of acidic nonsteroidal antiinflammatory drugs.**

(57) Quaternary ammonium alkyl esters and thioesters of acidic nonsteroidal anti-inflammatory drugs (NSAIDs) are disclosed. These esters and thioesters display the anti-inflammatory profile of the parent NSAIDs with greatly reduced gastrointestinal irritancy, providing a more favorable separation of therapeutic activity and toxicological side effects than the parent NSAIDs.

EP 0 289 262 A2

## Description

## ω-QUATERNARY AMMONIUM ALKYL ESTERS AND THIOESTERS OF ACIDIC NONSTEROIDAL ANTIINFLAMMATORY DRUGS

In recent years nonsteroidal antiinflammatory drugs (NSAID's) have been prescribed as the preferred therapy for chronic sufferers of arthritis and other inflammatory diseases. The major side-effects associated with chronic use of such drugs are gastrointestinal irritation and toxicity.

In particular, the salicylates, such as acetylsalicylic acid (aspirin), have been prescribed at high doses as the initial choice of drug therapy for the rheumatic diseases, such as rheumatoid arthritis. Unfortunately, serious adverse reactions can result from such high doses in some patients. For example, gastric damage is a prevalent side effect, such that ingestion of 1 to 3 grams per day of aspirin generally produces fecal blood loss of 2 to 6 ml/day in humans. Other adverse side effects include gastric ulcer formation, upper gastrointestinal hemorrhage allergic reactions, and even deafness. (See, Nonsteroidal Antiinflammatory Drugs, Lombardino, Ed., Chapter 4, Lombardino, p.262, John Wiley &; Sons, Inc., 1985.)

In attempts to diminish some of these side effects, non-salicylate classes of nonsteroidal anti-inflammatory drugs have been developed, such as the arylacetic acids, arylpropionic acids, fenamates and pyrrole carboxylic acids. While these newer classes of NSAIDs have achieved substantial therapeutic utility as antiinflammatory drugs for prolonged treatment of chronic diseases such as rheumatoid arthritis and osteoarthritis, they, too, tend to produce gastrointestinal side effects at high doses or with prolonged usage, impeding achievement of the desired therapeutic effect. These gastrointestinal side effects include minor gastric discomfort, nausea and dyspepsia through mucosal irritation and erosion with accompanying loss of blood into the gastrointestinal tract, and may also include such severe effects as ulcer formation, perforation of the gastrointestinal tract, and even death due to hemorrhage or septicemia.

For the weaker NSAIDs, such as aspirin and other salicylic acid derivatives (e.g., diflunisal), the gastrointestinal side effects appear to be caused by direct contact of undissolved or highly concentrated solutions of the drug with the mucosal cell lining of the gastrointestinal tract, leading to cell death and sloughing. In order to circumvent the effects of this undesirable direct contact, such drugs have been converted to certain esters which are well-tolerated by the gastrointestinal mucosal lining in spite of direct contact, and which are converted to the active drug by hydrolytic processes upon absorption. Such esters, generally being less soluble in the gastrointestinal tract than the parent compounds from which they are derived, are less well absorbed and most therefore be administered in substantially higher, frequently unrealistic, doses. In certain cases, however, a therapeutically effective dose appears to be achievable without the undesirable gastrointestinal side effects. For example, benorylate, the acetominophen ester of aspirin, has little if any adverse effects on the gastrointestinal tract, but suffers from the adverse toxic effects associated with salicylate drugs in general and the liver toxicity associated with acetaminophen in particular.

For more potent NSAIDs, such as the arylacetic and arylpropionic acids of which indomethacin and naproxen are typical, it has been found that the therapeutic to side effect (gastrointestinal irritation) ratio is substantially the same for both subcutaneous and oral administration of a given drug. It has been suggested that the adverse effects of these more potent compounds are not dependent upon direct contact of the substance in undissolved or in highly concentrated form with the gastrointestinal cells, but rather from therapeutically effective, circulatory plasma levels of these agents. Accordingly, the art has suggested (Nonsteroidal Antiinflammatory Drugs, Lombardino, Ed., Chapter 4, Otterness and Bliven, pp. 229-230,) that esters of such more potent substances should not be free of the anticipated gastrointestinal side effects associated with the parent compound, if such agents are administered in doses sufficient to provide therapeutically effective plasma levels of the parent drug as a consequence of hydrolysis of the ester to the parent compound. Previous experience with simple alkyl esters of NSAIDs proves this teaching to be generally correct. Simple alkyl esters, being less soluble, are less readily absorbed and demonstrably less readily hydrolyzed, (See, Bundgaard and Nielsen, Communication, J. Med. Chem., 30 (3): 451-454 (1987)), and therefore their improved therapeutic-to- side-effect ratio is more an artifact of modelling and timing of administration than a clinical reality.

Thus, a need exists for NSAIDs that have good therapeutic activity, but that are protective or less irritating to the gastrointestinal tract, and that inhibit formation of ulcers or are less likely to cause them.

A first aspect of this invention relates to compounds of the formula:

$$RC(O)M(CH_2)_nN^+R^1R^2R^3\ X^- \quad (I)$$

wherein:

R is a radical such that RCOOH is an acidic nonsteroidal antiinflammatory drug (ANSAID);

M is O of S;

n is an integer from 2 to 6;

$R^1$ and $R^2$ are independently alkyl of 1 to 6 carbon atoms having a $-CH_2-$ group directly connected to the nitrogen atom, phenyl, or benzyl;

or $R^1$ and $R^2$ may be combined with the nitrogen atom to which they are attached to form morpholino or a 5- or 6- membered saturated heterocyclic ring;

$R^3$ is alkyl of 1 to 6 carbon atoms having a $-CH_2-$ group directly connected to the nitrogen atom, or benzyl; and X is a pharmaceutically acceptable anion.

Another aspect of this invention relates to a method of treating inflammation in a mammal, such as a human, comprising the step of administering a therapeutically effective amount of a compound of Formula I to said mammal.

Yet another aspect of the invention relates to a pharmaceutical composition comprising a therapeutically effective amount of a compound of Formula I in admixture with a pharmaceutically acceptable excipient.

A still further aspect of the invention relates to a composition of matter comprising a therapeutically effective combined amount of an admixture of a compound of Formula I and a nonsteroidal antiinflammatory drug.

Still another aspect of the invention relates to a pharmaceutical composition comprising a therapeutically effective combined amount of a compound of Formula I in admixture with a nonsteroidal antiinflammatory drug and a pharmaceutically acceptable excipient.

This invention is directed to novel ω-quaternary ammonium alkyl esters and thioesters of acidic non-steroidal anti-inflammatory drugs. These esters show slightly reduced antiinflammatory activity but dramatically reduced intestinal ulcerogenic activity relative to the parent carboxylic acids.

Definitions

When used in this specification and the appended claims, the following terms are intended to have the meanings set forth below.

"Acidic nonsteroidal anti-inflammatory drugs," or ANSAIDs, are: non-salicylates having a free carboxylic acid moiety (i.e., of the formula RCOOH); having anti-inflammatory activity in mammals, particulary in humans, i,.e., activity in at least one accepted antiinflammatory or analgesic assay, such as the carrageenan paw assay, the phenylbenzoquinone writhing assay, or the adjuvant arthritis assay; and that are publicly known, commercially available, and/or whose synthesis is known or apparent to one of ordinary skill in the art of organic chemistry. Some ANSAIDs have one or more chiral centers, where one or more isomers are therapeutically active. The term ANSAID in accordance with this definition means the therapeutically active isomers, whether in resolved form (i.e., pure therapeutically active isomer), or as a mixture of isomers (i.e., any combination of therapeutically active enantiomers or diastereomers).

ANSAIDs include, but are not limited to, those carboxylic acids of the acetic acid, propionic acid and fenamate classes listed on pp. 254-255 of Nonsteroidal Antiinflammatory Drugs, Lombardino, Ed., Chapter 4: "Medicinal Chemistry of Nonsteroidal Antiinflammatory Drugs," by Lombardino, pp. 253-431 (John Wiley and Sons, Inc. 1985). The compounds named in the Lombardino text are representative of the most popularly accepted and widely used antiinflammatory agents. This text is hereby fully incorporated into this specification by reference. Specifically included are: the acetic acids - indomethacin (U.S. Pat. No. 3,161,654), acemetacin, cinmetacin, sulindac (U.S. Pat. No. 3,654,349), tolmetin (U.S. Pat. No. 3,752,826), zomepirac (U.S. Pat. No. 3,752,826), diclofenac, fenclofenac, isoxepac, furofenac, fentiazac, clidanac, oxepinac, fenclorac, lonazolac, metiazinic acid, clopirac, amfenac, benzofenac, clometacine, etodolac, bumidazone, and clamidoxic acid; the propionic acids - ibuprofen (U.S. Pat. No. 3,385,886), flurbiprofen, naproxen (U.S. Pat. No. 3,904,682), ketoprofen, fenoprofen (U.S. Pat. No. 3,600,437), benoxaprofen (U.S. Pat. Nos. 2,912,748 and Re. 29,608), indoprofen, carprofen, oxaprozin, pranoprofen, suprofen, miroprofen, tioxaprofen, alminoprofen, cicloprofen, tiaprofenic acid, furaprofen, butibufen, fenbufen, bucloxic acid and protizinic acid; and the fenamates - mefenamic acid (U.S. Pat. No. 3,138,636), flufenamic acid, meclofenamate (U.S. Pat. No. 3,313,848), niflumic acid, tolfenamic acid, flunixin and clonixin. In addition, compound related to the above drugs are included within this ANSAID definition, such as those families of compound disclosed in U.S. patents encompassing the specific drugs listed herein. The syntheses of the foregoing ANSAIDs are known and set forth in Chapter 4 of the Lombardino text cited above, and incorporated herein by reference.

In addition, ANSAIDs include the pyrrolo-pyrrole carboxylic acid classes of drugs, disclosed in: U.S. Pat. No. 4,089,969 to Muchowski and Kluge, issued May 16, 1978 (i.e., 5-aroyl-1,2-dihydro-3H-pyrrolo[1,2-a]pyrrole-1-carboxylic acid derivatives), such as ketorolac (5-benzoyl-...) and anirolac (5-p-anisoyl-...); U.S. Pat. No. 4,097,579 to Muchowski and Kluge, issued June 27, 1978 (i.e., 5-(2-pyrroyl)-1,2-dihydro-3H-pyrrolo[1,2a]pyrrole-1-carboxylic acid derivatives); and (U.S. Pat. No. 4,087,539 to Muchowski and Kluge, issued May 2, 1978 (i.e., 5-(2-furoyl)-, 5-(2-thenoyl)-, 5-(3-furoyl)- and 5-(3-thenoyl)-1,2-dihydro-3H-pyrrolo[1,2a]pyrrole-1-carboxylic acid derivatives). The foregoing patents are hereby fully incorporated into this specification by reference.

Further, the term ANSAID as used herein includes the acetic acid, tifurac, having the chemical name, 7-(4-methylthiobenzoyl)benzofuran-5-ylacetic acid. This compound is disclosed in EPO Application Publication No. 0 132 130, published January 22, 1986, said publication being fully incorporated into the instant specification by reference.

Additionally, ANSAIDS include those compounds related to naproxen as well as naproxen (2-(6′-methoxy-2′-naphthyl)propionic acid, that are disclosed in (U.S. Pat. No. 3,904,682 issued to Fried et al. on September 9, 1975. (U.S. Pat. No. 3,904,682 is hereby fully incorporated into this specification by reference.

"Alkyl" means a radical consisting of a straight or branched chain of carbon and hydrogen atoms. "Alkyl of 1 to 6 carbon atoms having a $-CH_2-$ group directly connected to the nitrogen atom" includes methyl, ethyl, n-propyl, iso-propyl, n-butyl, iso-butyl, sec-butyl, n-pentyl, iso-pentyl, neo-pentyl, n-hexyl, isohexyl, and all isomers thereof lacking branching of the chain of the carbon atom directly attached to the ammonium

nitrogen; specifically excluded are tert-butyl (2,2-dimethylethyl), tert-pentyl (2,2-dimethylpropyl), and tert-hexyl (2,2-dimethylbutyl). "Lower alkyl" and "alkyl of 1 to 4 carbon atoms" are synonymous as used herein, and are coextensive with the definition of alkyl of 1 to 6 carbon atoms having a $-CH_2-$ group directly connected to the nitrogen atom, except that the number of carbon atoms is limited to four.

"Halo" is iodo, bromo or chloro.

A "saturated heterocyclic ring" of 5 or 6 members referred to herein is a saturated radical of carbon and hydrogen atoms, together with the nitrogen atom of the quaternary ammonium compound of Formula I, arranged in a ring structure, such as piperidinyl or pyrrolidinyl.

"Nonsteroidal anti-inflammatory drugs," or NSAIDs, include all classes of anti-inflammatory drugs that have anti-inflammatory or analgesic activity, ie., activity in at least one accepted antiinflammatory or analgesic assay, such as the carrageenan paw assay, the phenylbenzoquinone writhing assay, or the adjuvant arthritis assay; and that are publicly known, commercially available, and/or whose synthesis is known or apparent to one of ordinary skill in the art of organic chemistry.

A "pharmaceutically acceptable anion" is an anion that is not biologically or otherwise undesirable, and that either results from the alkylation step of the process for making the compounds of Formula I, or can be interchanged with another anion (or series of anions) ultimately originating from such an alkylation step. Examples of such pharmaceutically acceptable anions are halo, tosylate, mesylate, phosphate, acetate or succinate.

"Salicylates" are those drugs defined as such in the Lombardino text (cited above), i.e., acetylsalicylic acid, salsalate, diflunisal and fendosal. Non-salicylates are NSAIDs excluding these compounds.

Within the broad scope of the invention, certain embodiments of the compounds of Formula I are specifically preferred. These embodiments are also preferred for the aspects of the invention relating to pharmacological compositions and methods of use.

Thus, while any ANSAID corresponding to RCOOH can provide the RC(O)- radical of the claims, the preferred compounds are indomethacin, sulindac, tolmetin, tifurac, zomepirac, diclofenac, fenclofenac, etodolac, ibuprofen, naproxen, ketoprofen, benoxaprofen, mefenamic acid, flufenamic acid, ketorolac and anirolac. Of these, more preferred are naproxen, ketorolac, anirolac, diclofenac, tifurac, ibuprofen, indomethacin, and sulindac. Of these, the most preferred are naproxen, ketorolac, anirolac, tifurac and indomethacin.

Likewise, ther are certain preferred substituents for $R^1$, $R^2$ and $R^3$, whether for the broad invention or the more preferred aspects. Generally, these substituents are all preferably alkyl of 1 to 6 carbon atoms having a $-CH_2-$ group directly connected to the ammonium nitrogen; more preferably, lower alkyl (1 to 4 carbon atoms); and most preferably, methyl.

The pharmaceutically acceptable anion X is preferably halo, and most preferably chloro. However, in some cases, iodide is currently preferred; for example, naproxen thiocholine iodide is currently a most preferred compound.

The number of methylene groups is preferably 2 or 3, and most preferably 2.

Finally, M is generally preferably S.

Particularly preferred compounds are naproxen thiocholine chloride, naproxen thiocholine bromide and naproxen thiocholine iodide.

Preferred utilities and modes of administration are discussed below.

## Utility

The compounds of Formula I have a number of utilities. They can be used singly as therapeutic agents in the treatment of inflammatory diseases or inflammatory conditions of the muscular skeletal system, skeletal joints and other tissues, such as arthritis (rheumatoid arthritis, osteoarthritis, etc.), concussion, laceration, sports injuries bone fractures, post-traumatic conditions, gout, dysmenorrhea, migraine and multiple sclerosis. Alternatively or in addition, the compounds of Formula I can be used in conjunction with other NSAIDs or ANSAIDs. By combining a compound of Formula I with a therapeutically effective dose of an ANSAID or NSAID, the gastrointestinal irritation resulting from administration of the ANSAID or NSAID can be reduced.

The compounds of Formula I are particularly suitable for use in arthritis therapy, either alone or in combination with another anti-inflammatory or analgesic compound. In the latter event, it is particularly preferred that the compound of Formula I be co-administered with the corresponding ANSAID, i.e, that the RC(O)-portion of the compound of Formula I be the same as that portion of the co-administered ANSAID.

## Dosage and Administration

Administration of the compounds of Formula I will either be by "solo-administration," i.e., administration of said compounds as the sole anti-inflammatory or analgesic agent in the therapy; or will be by "co-administration," i.e., administration with another NSAID or with an ANSAID such that the compound of Formula I protects the gastrointestinal tract from the extent of erosion and/or ulceration that would otherwise occur.

The compounds of this invention are administered to a mammal or human in need thereof in a therapeutically effective dose. Such a dose is either: (1) in the case of solo-administration, an amount sufficient to treat or alleviate the symptoms of the disease state, i.e. of inflammatory conditions such as arthritis (rheumatoid arthritis, osteoarthritis, etc.), sports injuries, etc., or (2) in the case of co-administration, an amount sufficient

to inhibit the amount of gastrointestinal erosion and/or ulceration that would otherwise take place.

The weight amount of active compound administered will of course, be dependent on the subject being treated, the nature and severity of the affliction, the molecular weight of the active ingredient, the manner of administration and the judgment of the prescribing physician. However, an effective dosage for a human is generally, for either solo-administration or co-administration, in the range of about 0.001 to about 250 mg/kg/day, preferably about 0.1 to about 150 mg/kg/day, and most preferably about 0.5 to about 50 mg/kg/day. More precise dosages for a given set of circumstances will readily be ascertainable by one of ordinary skill in the art when taking into consideration generally prescribed dosage ranges for the particular parent ANSAID. Accordingly, these concentrations are provided only as a general guide and should not be construed as limiting the scope of the invention. In general, a suitable dose will be selected to provide a therapeutically effective antiinflammatory dose without adverse effects on the mucosa of the gastrointestinal tract (bleeding, erosion, ulceration, perforation, etc.).

Administration of the active compounds and salts described herein can be via any of the accepted modes of administration for agents which have the intended therapeutic use. These methods include oral, parenteral, topical, transdermal and otherwise systemic or aerosol forms.

Depending on the intended mode of administration, the compositions used may be in the form of solid, semi-solid or liquid dosage forms, such as, for example, tablets, suppositories, pills, capsules, powders, liquids, suspensions, or the like, preferably in unit dosage forms suitable for singe administration of precise dosages. The compositions will include a conventional pharmaceutical carrier or excipient and an active compound of Formula I or the pharmaceutically acceptable salts thereof and, in addition, may include other medicinal agents, pharmaceutical agents, carriers, adjuvants, etc.

For oral administration, a pharmaceutically acceptable non-toxic composition is formed by the incorporation of any of the normally employed excipients, such as, for example pharmaceutical grades of mannitol, lactose, starch, magnesium stearate, sodium saccharin, talcum, cellulose, glucose, sucrose, magnesium carbonate, and the like. Such compositions take the form of solutions, suspensions, tablets, pills, capsules, powders, sustained release formulations and the like. Such compositions may contain 10%-95% active ingredient, preferably 25-70%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, etc.

For systemic administration via suppository, traditional binders and carriers include, e.g. polyalkylene glycols or triglycerides. Such suppositories may be found from mixtures containing active ingredient in the range of 0.5%-10%; preferably 1-2%.

PREPARATION AND EXAMPLES

Compounds of the present invention can be made directly and most conveniently from the parent acidic nonsteroidal antiinflammatory agent (ANSAID). In summary, the ANSAID is activated at the carboxyl group of the acid functionality by a variety of reagents. The resulting activated ANSAID derivative is then condensed with an alcohol or alkyl thiol possessing an ω-dialkylamino functionality. The resulting alkyl(thio) ester is then subjected to quaternization with an alkylating agent to afford the title compounds.

Compounds of the present invention are prepared by the reaction sequence outlined in Reaction Scheme A.

## REACTION SCHEME A

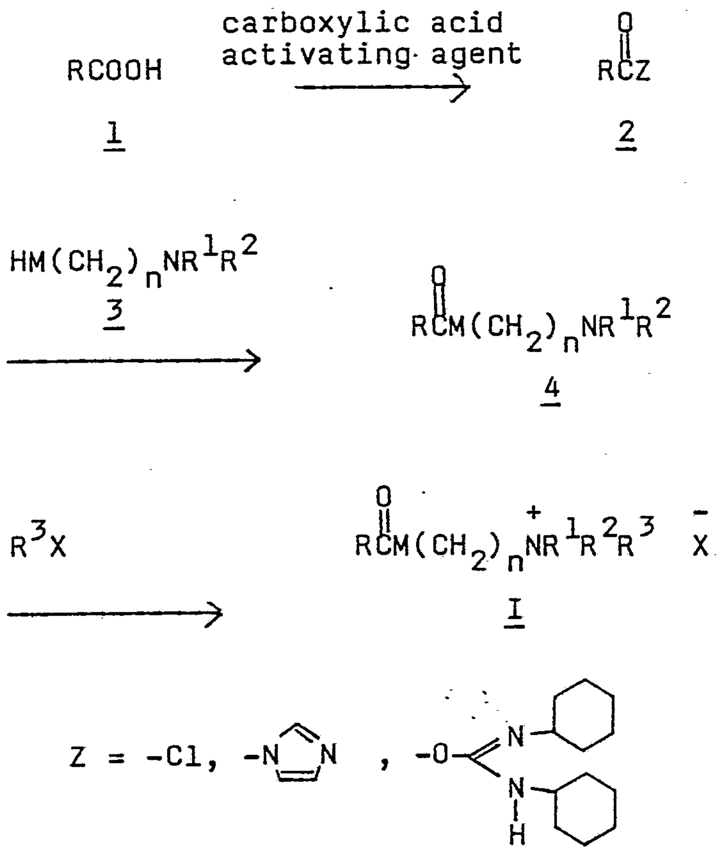


A Reaction Scheme A, the acidic nonsteroidal antiinflammatory drugs of the formula RCOOH (formula 1) are readily available from commercial sources, e.g., Sigma Chemical Corp., or are readily prepared by the methods described in the art generally, or are prepared by synthetic methods incorporated herein by reference (Lombardino, Chapter 4, cited above, and incorporated by reference herein specifically for methods of making the various ANSAIDS disclosed therein). The various ANSAIDs are converted to active acylating agents (formula 2) by any number of reagents, such as thionyl chloride, oxalyl chloride, 1,1′-carbonyldiimidazole, N,N′-dicyclohexylcarbodiimide, or other commercially available acid activating reagents. The reagent of choice is 1,1′-carbonyldiimidazole. Generally, the reaction is carried out by addition of a mole equivalent, or up to a 25% mole excess of the reagent with the ANSAID, in an aprotic solvent such as benzene, tetrahydrofuran, diethyl ether or dichloromethane, preferably tetrahydrofuran, at temperatures between 0°C and the reflux temperature of the solvent, but preferably at room temperature, for a period of 1 to 16 hours, but preferably for 4 hours.

To the solution of active acylating agent (formula 2) is added a solution prepared from 1 to 1.5 mole equivalents of the desired alcohol or thiol (formula 3) which has been treated with from 0.1 to 1.0 equivalents of an alkyl lithium reagent, such as n-butyllithium. When compounds of formula 3 are supplied as acid addition salts, such as the hydrochloride salt, an additional equivalent of the alkyl lithium base must be added to the preformed solution of formula 3 added to the solution of the active acylating agent (formula 2). The solvent for this mixture is preferably identical to that used to prepare the solution of the compound of formula 2. The resulting reaction mixture is stirred at temperatures between ambient and the reflux temperature of the solvent, but preferably at ambient temperature, for a period of 1 to 16 hours, preferably 12 hours. The solvent is removed by evaporation, and the resulting residue is subjected to aqueous extraction to afford esters of the formula 4.

The esters of formula 4 are dissolved in an aprotic solvent such as diethyl ether, tetrahydrofuran, ethyl acetate and the like, but preferably diethyl ether, and are treated with 1 to 3 equivalents of an alkylating agent of the formula $R^3X$ such as alkyl halides, alkyl sulfonates, at temperatures from ambient to the reflux temperature of the solvent, but preferably at ambient temperature, or alternatively in a sealed pressure apparatus without solvent, for a period of 12 to 72 hours, preferably 24 hours. The desired compounds of Formula I are recovered from the reaction mixture by filtration, and may be further purified by recrystallization from solvents such as

acetone, acetonitrile and the like.

The required alcohols or thiols of the formula 3 are available from chemical suppliers, e.g., Aldrich Chemical Co. Compounds of formula 3 that are not readily commercially available may be made according to procedures described in the chemical literature by one skilled in the art. In particular, compounds of formula 3 where n = 2 may be prepared by the reaction of a secondary amine $R^1R^2NH$ with either ethylene oxide, as described in J. Am. Chem. Soc., 66, 1640 (1944), or ethylene sulfide, as described in J. Am. Chem. Soc., 69, 2672 (1947) and graphically depicted in Reaction Scheme B. In either case, the desired secondary amine is treated with an excess of the appropriate ethylene chalcogenide, in polar solvents such as methanol, ethanol or N,N-dimethylformamide, methanol being preferred. Alternatively, especially in the case of ethylene sulfide, the reactants are combined neat, and heated in a pressure apparatus to temperatures from about 50 to about 120° C, but preferably around 100° C, for about 2 to about 24 hours, preferably around 18 hours. Evaporation of the solvent, if used, followed by distillation and/or crystallization of the acid addition salt of the product affords the desired product of formula 3.

REACTION SCHEME B

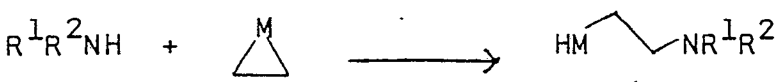

3 (n = 2,
M = O or S)

Compounds of formula 3 where M = O and n is greater than 2 but not available commercially may be prepared by reaction of commercially available ω-haloalcohols with 1 to 3 equivalents of a secondary amine, by the method described in J. Am. Chem. Soc., 66, 1640 (1944), graphically depicted in Reaction Scheme C. A mixture of the ω-haloalcohol and from 1 to 3 equivalents of the desired secondary amine is heated at temperatures from about 50 to about 120°C, preferably around 100°C, in a sealed pressure apparatus if necessary, for a period of 2 hours to 72 hours, around 48 hours being preferred. The reaction is complete when the haloalcohol is consumed. Fractionation of the reaction mixture by distillation, aqueous extraction or fractional crystallization of the acid addition salt of the product away from that of excess reactant allow isolation of the desired 3.

REACTION SCHEME C

$$HO(CH_2)_nY + R^1R^2NH \longrightarrow HO(CH_2)_nNR^1R^2$$

Y = -halo

3 (M = O)

Compounds of formula 3 where M = S and n is greater than 2 may be prepared from the corresponding compound where M = O by conversion of the alcohol to the corresponding tosylate using tosyl chloride in about a 10% mole excess in solvents such as tetrahydrofuran, chloroform or dichloromethane, with the last being preferred. After evaporation of the solvent, the resulting ω-tosyloxy amine hydrochloride (formula 5) is directly treated with from 1 to 3 molar equivalents of potassium ethyl xanthate in a dipolar aprotic solvent such as tetrahydrofuran or N,N-dimethylformamide, the latter being preferred, at temperatures from ambient to 60°C, room temperature being preferred. After removal of the solvent the resulting xanthate (formula 6) is hydrolysed using a strong base, such as ammonium hydroxide or sodium hydroxide, to afford the desired compound of formula 3 where M = S, and recovered from the reaction mixture by conventional means, such as aqueous extraction, distillation, or conversion to an acid addition salt such as the hydrochloride. The process is graphically depicted in Reaction Scheme D.

## REACTION SCHEME D

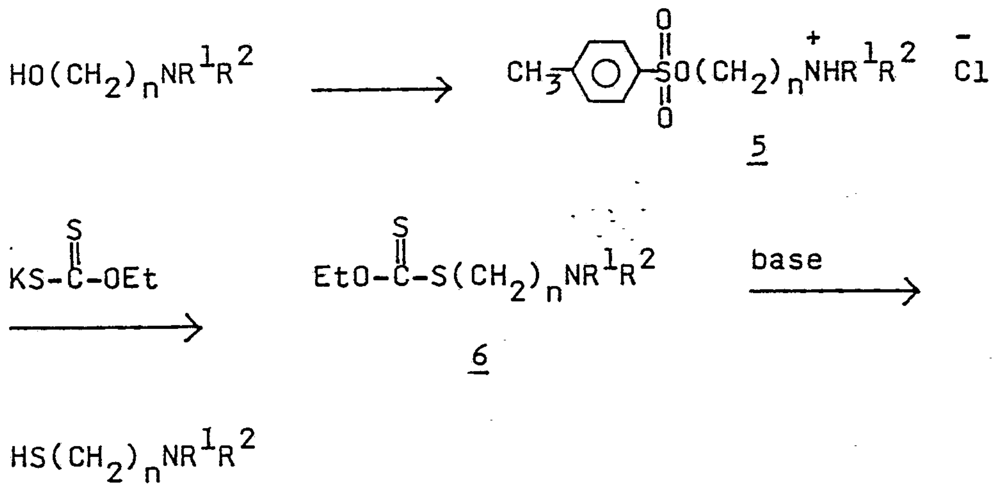


5

6

3 (M = S)

The following specific examples are intended to teach and illustrate various aspects of the invention. However, the claims, and all equivalents thereof, provide the metes and bounds of the scope of the invention.

ALTERNATIVE SYNTHETIC ROUTES

In addition to the Reaction Schemes set forth above, there are other methodologies that can be used to make the compounds of Formula I. For example:

Alternative 1:

Quaternization of an ω-haloalkyl ester of an ANSAID

Alternatively, as shown in Scheme E, an ANSAID may be converted to its corresponding ω-haloalkyl ester (formula A) by any number of methods, including but not limited to acid-catalysed esterification or condensation of the corresponding acid chloride with the alcohol of choice. The resulting ω-haloalkyl ester of formula A is subsequently treated with an amine of the formula $R^1R^2R^3N$ to afford compounds of Formula I, where M = O.

## REACTION SCHEME E

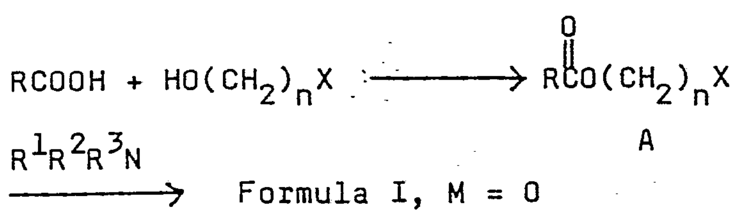

A

Formula I, M = O

For exemplification of this alternative, see Preparation 5 (for formula A) and Example 3 (for Formula I).

Alternative 2:

Direct esrterification of an ANSAID with Choline or Thiocholine

Alternatively, an ANSAID or a derivative thereof can be directly esterified to form the desired end product of Formula I. The derivative can be acyl halides, acidic anhydrides and the like. As outlined in Scheme F, an activated acylating agent derived from an ANSAID described above, preferably the acid chloride, is treated directly with a choline or thiocholine halo salt, such as chloline chloride, thiocholine chloride or the like, in the presence of an equivalent of tertiary amine base, in solvents such as tetrahydrofuran, acetonitrile or

dimethylformamide, at room temperature to 50° C, to directly afford compounds of Formula I with $R^1$, $R^2$ and $R^3$ defined as above after evaporation of the solvent and recrystallization.

REACTION SCHEME F

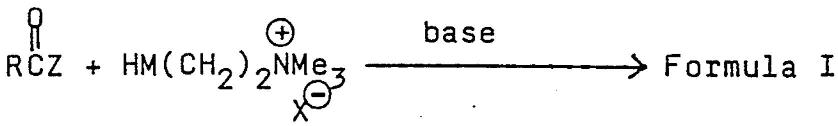


Z = Cl or as previously defined above

For exemplification of this alternative, see Example 4 (for Formula I).

Alternative 3:

A third alternative method for obtaining the compounds of Formula I is the acid-catalyzed transesterification of an ANSAID alkyl ester, e.g., methyl or ethyl, with a choline or thiocholine salt. The acid anion is preferably the same as the anion of the salt used, i.e., HCl for the chloride salt.

PREPARATION 1

N,N-DIBENZYLETHANOLAMINE AND RELATED COMPOUNDS OF FORMULA (3) BY REACTION SCHEME (B)

A. Condensed ethylene oxide (7 ml, 125 mmol) was added to a mixture of dibenzylamine (19.2 ml, 100 mmol) in methanol (20 ml) cooled to 0° C. After 2 hours at room temperature, the mixture was evaporated without heating above room temperature. The residue was distilled to afford the title compound, bp. 220-225° C/25 mm, which solidified on standing, mp 48° C.

B. Similarly prepared by use of the appropriate secondary amines are the following N,N-disubstituted ethanolamines:

2-(1-piperidinyl)ethanol;
2-(1-pyrrolidinyl)ethanol;
2-(4-morpholinyl)ethanol;
N,N-di-n-butylethanolamine;
N,N-diphenylethanolamine;
N,N-dimethylethanolamine;
N,N-diethylethanolamine;
N,N-di-n-propylethanolamine;
N-methyl-N-benzylethanolamine;
N-phenyl-N-benzylethanolamine;
N-ethyl-N-butylethanolamine; and
N-phenyl-N-ethylethanolamine.

PREPARATION 2

2-(1-PIPERIDINYL)ETHANETHIOL AND RELATED COMPOUNDS OF FORMULA (3) BY REACTION SCHEME (B)

A. A mixture of ethylene sulfide (15 ml, 250 mmol) and piperidine (25 ml, 250 mmol) was heated at 100° C in a sealed pressure tube for 18 hours. After cooling, the mixture was distilled to afford the title compound, bp. 55-58° C/4 mm.

B. Similarly prepared by use of the appropriate secondary amines are the following N,N-disubstituted aminoethanethiols:

2-(1-pyrrolidinyl)ethanethiol;
2-(4-morpholinyl)ethanethiol;
N,N-di-n-butylaminoethanethiol;
N,N-dibenzylaminoethanethiol;
N,N-diphenylaminoethanethiol;
N,N-dimethylaminoethanethiol;
N,N-diethylaminoethanethiol;
N,N-di-n-propylaminoethanethiol;
N,N-di-n-butylaminoethanethiol;
N-methyl-N-benzylaminoethanethiol;

N-phenyl-N-benzylaminoethanethiol;
N-ethyl-n-butylaminoethanethiol; and
N-phenyl-N-ethylaminoethanethiol.

PREPARATION 3

4-(N,N-DIPROPYLAMINO)BUTANOL AND RELATED COMPOUNDS OF FORMULA (3) BY REACTION SCHEME (C)

A. A mixture of 4-chloro-1-butanol (20 ml, 200 mmol) and dipropylamine (68 ml, 500 mmol) was heated to 100°C under a blanket of nitrogen for 48 hours. Fractional distillation afforded the title compound as a colorless liquid, bp 100°C/16mm.

B. Similarly prepared by use of the appropriate secondary amines and the appropriate ω-haloalcohols are the following N,N-disubstituted aminoalcohols:
4-(4-morpholinyl)butanol;
4-(1-piperidinyl)butanol;
4-(1-pyrrolidinyl)butanol;
4-(N,N-diphenylamino)butanol;
4-(N,N-dimethylamino)butanol;
4-(N,N-diethylamino)butanol;
4-(N,N-di-isopropylamino)butanol;
4-(N,N-di-n-butylamino)butanol;
4-(N-methyl-N-benzylamino)butanol;
4-(N-phenyl-N-benzylamino)butanol;
4-(N-ethyl-N-butylamino)butanol;
4-(N-phenyl-N-ethylamino)butanol;
3-(4-morpholinyl)propanol;
3-(1-piperidinyl)propanol;
3-(1-pyrrolidinyl)propanol;
3-(N,N-di-isopropylamino)propanol;
3-(N,N-diphenylamino)propanol;
3-(N,N-dimethylamino)propanol;
3-(N,N-diethylamino)propanol;
3-(N,N-di-n-propylamino)propanol;
3-(N,N-di-n-butylamino)propanol;
3-(N-methyl-N-benzylamino)propanol;
3-(N-phenyl-N-benzylamino)propanol;
3-(N-ethyl-N-butylamino)propanol;
3-(N-phenyl-N-ethylamino)propanol;
3-(N,N-diphenylamino)propanol;
3-(N,N-dimethylamino)propanol;
3-(N,N-diethylamino)propanol;
3-(N,N-di-n-propylamino)propanol;
3-(N,N-di-n-butylamino)propanol;
3-(N-methyl-N-benzylamino)propanol;
3-(N-phenyl-N-benzylamino)propanol;
3-(N-ethyl-N-butylamino)propanol;
3-(N-phenyl-N-ethylamino)propanol;
5-(4-morpholinyl)pentanol;
5-(1-piperidinyl)pentanol;
5-(1-pyrrolidinyl)pentanol;
5-(N,N-di-isopropylamino)pentanol;
5-(N,N-diphenylamino)pentanol;
5-(N,N-dimethylamino)pentanol;
5-(N,N-diethylamino)pentanol;
5-(N,N-di-n-propylamino)pentanol;
5-(N,N-di-n-butylamino)pentanol;
5-(N-methyl-N-benzylamino)pentanol;
5-(N-phenyl-N-benzylamino)pentanol;
5-(N-ethyl-N-butylamino)pentanol;
5-(N-phenyl-N-ethylamino)pentanol;
6-(4-morpholinyl)hexanol;
6-(1-piperidinyl)hexanol;
6-(1-pyrrolidinyl)hexanol;
6-(N,N-di-isopropylamino)hexanol;
6-(N,N-diphenylamino)hexanol;

6-(N,N-dimethylamino)hexanol;
6-(N,N-diethylamino)hexanol;
6-(N,N-di-n-propylamino)hexanol;
6-(N,N-di-n-butylamino)hexanol;
6-(N-methyl-N-benzylamino)hexanol;
6-(N-phenyl-N-benzylamino)hexanol;
6-(N-ethyl-N-butylamino)hexanol; and
6-(N-phenyl-N-ethylamino)hexanol.

PREPARATION 4

4-(N,N-DIETHYLAMINO)BUTANETHIOL AND RELATED COMPOUNDS OF FORMULA (3) BY REACTION SCHEME (C)

A. A chilled dichloromethane solution of 4-(diethylamino)butanol, bp 86-87°C/25 mm (29 g, 200 mmol) prepared as in Preparation 3, was treated portionwise with p-toluenesulfonyl chloride (42 g, 220 mmol) at a rate such that the temperature never exceeded 20°C. After stirring 1 hour at room temperature, the solvent was removed by evaporation, and the residue was dissolved in N,N-dimethylformamide (100 ml). To this solution was added potassium ethyl xanthate (80 g, 500 mmol) in small portions. After stirring overnight at 60°C, the reaction mixture was partitioned between diethyl ether and water (200 ml each). The organic layer was washed with water (3 × 100 ml) and with brine (2 × 100 ml), and was then dried over sodium sulfate, filtered and evaporated. The residue was treated with 10% aqueous sodium hydroxide overnight, and the resulting solution was extracted with ether (4 × 100 ml). The organic extract was dried as above, filtered and evaporated. Fractional distillation afforded the title compound, bp. 95°C/15 mm.

B. Similarly prepared from the ω-disubstituted aminoalcohols of Preparation 3 are the following thiols:
4-(4-morpholinyl)butanethiol;
4-(1-piperidinyl)butanethiol;
4-(1-pyrrolidinyl)butanethiol;
4-(N,N-diphenylamino)butanethiol;
4-(N,N-dimethylamino)butanethiol;
4-(N,N-di-isopropylamino)butanethiol;
4-(N,N-di-n-propylamino)butanethiol;
4-(N,N-di-n-butylamino)butanethiol;
4-(N-methyl-N-benzylamino)butanethiol;
4-(N-phenyl-N-benzylamino)butanethiol;
4-(N-ethyl-N-butylamino)butanethiol;
4-(N-phenyl-N-ethylamino)butanethiol;
3-(4-morpholinyl)propanethiol;
3-(1-piperidinyl)propanethiol;
3-(1-pyrrolidinyl)propanethiol;
3-(N,N-di-isopropylamino)propanethiol;
3-(N,N-diphenylamino)propanethiol;
3-(N,N-dimethylamino)propanethiol;
3-(N,N-diethylamino)propanethiol;
3-(N,N-di-n-propylamino)propanethiol;
3-(N,N-di-n-butylamino)propanethiol;
3-(N-methyl-N-benzylamino)propanethiol;
3-(N-phenyl-N-benzylamino)propanethiol;
3-(N-ethyl-N-butylamino)propanethiol;
3-(N-phenyl-N-ethylamino)propanethiol;
3-(N,N-diphenylamino)propanethiol;
3-(N,N-dimethylamino)propanethiol;
3-(N,N-diethylamino)propanethiol;
3-(N,N-di-n-propylamino)propanethiol;
3-(N,N-di-n-butylamino)propanethiol;
3-(N-methyl-N-benzylamino)propanethiol;
3-(N-phenyl-N-benzylamino)propanethiol;
3-(N-ethyl-N-butylamino)propanethiol;
3-(N-phenyl-N-ethylamino)propanethiol;
5-(4-morpholinyl)pentanethiol;
5-(1-piperidinyl)pentanethiol;
5-(1-pyrrolidinyl)pentanethiol;
5-(N,N-di-isopropylamino)pentanethiol;
5-(N,N-diphenylamino)pentanethiol;
5-(N,N-dimethylamino)pentanethiol;

5-(N,N-diethylamino)pentanethiol;
5-(N,N-di-n-propylamino)pentanethiol;
5-(N,N-di-n-butylamino)pentanethiol;
5-(N-methyl-N-benzylamino)pentanethiol;
5-(N-phenyl-N-benzylamino)pentanethiol;
5-(N-ethyl-N-butylamino)pentanethiol;
5-(N-phenyl-N-ethylamino)pentanethiol;
6-(4-morpholinyl)hexanethiol;
6-(1-piperidinyl)hexanethiol;
6-(1-pyrrolidinyl)hexanethiol;
6-(N,N-di-isopropylamino)hexanethiol;
6-(N,N-diphenylamino)hexanethiol;
6-(N,N-dimethylamino)hexanethiol;
6-(N,N-diethylamino)hexanethiol;
6-(N,N-di-n-propylamino)hexanethiol;
6-(N,N-di-n-butylamino)hexanethiol;
6-(N-methyl-N-benzylamino)hexanethiol;
6-(N-phenyl-N-benzylamino)hexanethiol;
6-(N-ethyl-N-butylamino)hexanethiol; and
6-(N-phenyl-N-ethylamino)hexanethiol.

PREPARATION 5

NAPROXEN 2-BROMOETHYL ESTER BY REACTION SCHEME E

A mixture of naproxen (11.5 g, 50 mmol), 2-bromoethanol (4.25 ml, 60 mmol) and p-toluenesulfonic acid (0.5 g) in toluene (100 ml) was heated to reflux overnight, with removal of water via a Dean-Stark apparatus. The mixture was cooled, washed with half-saturated aqueous sodium bicarbonate twice, and with brine twice. The organic extract was dried over sodium sulfate, filtered and evaporated to afford the title compound as an oil.

Similarly prepared by substitution of 2-chloroethanol, 2-iodoethanol, 3-bromopropanol and 4-bromobutanol, respectively are:
naproxen 2-chloroethyl ester;
naproxen 2-iodoethyl ester;
naproxen 3-bromopropyl ester; and
naproxen 4-bromobutyl ester.
Other ω-haloalcohols can be used in this preparation to make analogous compounds.

Similarly prepared by substitution of the corresponding ANSAID are:
ketorolac 2-bromoethyl ester;
ketorolac 2-chloroethyl ester;
ketorolac 2-iodoethyl ester;
indomethacin 3-bromopropyl ester;
sulindac 4-bromobutyl ester.
Other ω-haloalcohols can be used in this preparation to make analogous compounds.

EXAMPLE 1

NAPROXEN CHOLINE ESTER IODIDE AND RELATED ESTERS OF FORMULA I

A.0. A solution of naproxen (11.51 g, 50 mmol) in dry tetrahydrofuran (100 ml) was treated with solid 1,1′-carbonyldiimidazole (9.3g), and was stirred 1 hour at room temperature. To this solution was added a tetrahydrofuran solution prepared from 2-dimethylaminoethanol (7.5 ml) and n-butyllithium (5 ml of 1.6 M solution in hexane), and the resulting mixture was stirred at room temperature overnight. The solvent was removed by evaporation, and the residue was dissolved in diethyl ether and washed with water and brine. The organic extract was dried over sodium sulfate, and evaporated to afford an oil. The oily ester was dissolved in fresh diethyl ether and was treated with methyl iodide (9 ml). After stirring at ambient temperature overnight, the resulting precipitate was collected by filtration, washed with additional diethyl ether and dried in vacuo to afford naproxen choline ester iodide (18.5 g, 41.7 mmol, 83%) as a white crystalline solid, mp 194-195°C.

A.1. Similarly, by substituting ketorolac, indomethacin, sulindac, ibuprofen, zomepirac, flufenamic acid and ketoprofen, respectively, for naproxen, in the above example A.0, the following compounds of Formula I were prepared:
ketorolac choline ester iodide, mp. 80-82°C;
indomethacin choline ester iodide, mp. 209-210°C;
sulindac choline ester iodide, mp. 209-210°C;
ibuprofen choline ester iodide, mp. 127-128°C;
zomepirac choline ester iodide, mp. 170-172°C;
flufenamic acid choline ester iodide, mp. 138-139°C;
ketoprofen choline ester iodide, mp. 60-62°C;

mefenamic acid choline ester iodide, mp. 181-183° C;
and
etodolac choline ester iodide, mp. 104-106° C.

A.2. Similarly, by substituting benoxaprofen, etodolac, tolmetin, tifurac, diclofenac, anirolac, fenclofenac, and mefenamic acid, respectively, for naproxen, in the above example A.0, the following compounds of Formula I are prepared:
benoxaprofen choline ester iodide;
etodolac choline ester iodide, mp. 104-106° C;
tolmetin choline ester iodide;
tifurac choline ester iodide;
diclofenac choline ester iodide;
anirolac choline ester iodide; and
fenclofenac choline ester iodide.

B.1. Similarly, by optionally substituting other ANSAIDs (e.g., benoxaprofen, etodolac, tolmetin, etc. respectively - see A.1 and A.2 above) for naproxen and substituting other alkylating agents $R^3X$ for methyl iodide in the above example A.0, the following compounds of Formula I are prepared:
benoxaprofen choline ester chloride;
etodolac choline ester chloride;
tolmetin choline ester chloride;
tifurac choline ester chloride;
indomethacin choline ester chloride;
sulindac choline ester chloride;
zomepirac choline ester chloride;
diclofenac choline ester chloride;
fenclofenac choline ester chloride;
ibuprofen choline ester chloride;
naproxen choline ester chloride;
ketoprofen choline ester chloride;
mefenamic acid choline ester chloride;
flufenamic acid choline ester chloride;
diclofenac acid choline ester chloride;
ketorolac choline ester chloride;
anirolac choline ester chloride;
benoxaprofen choline ester bromide;
etodolac choline ester bromide;
tolmetin choline ester bromide;
tifurac choline ester bromide;
indomethacin choline ester bromide;
sulindac choline ester bromide;
zomepirac choline ester bromide;
diclofenac choline ester bromide;
fenclofenac choline ester bromide;
ibuprofen choline ester bromide;
naproxen choline ester bromide;
ketoprofen choline ester bromide;
mefenamic acid choline ester bromide;
flufenamic acid choline ester bromide;
diclofenac acid choline ester bromide;
ketorolac choline ester bromide; and
anirolac choline ester bromide.

B.2.a. Similarly, by substituting other ω-dialkylamino alcohols of formula (3) listed in (i) Preparation 1.B and (ii) Preparation 3.B above, for 2-dimethylaminoethanol, the following corresponding compounds of Formula I were prepared in accordance with the procedure described in this Example 1, section A.0, above:

(i) From the ω-dialkylamino alcohols of formula (3) listed in Preparation 1.B:
naproxen 2-(1-piperidinyl-N-methylammonium)ethyl ester iodide, mp. 114-115° C;
naproxen 2-(1-pyrrolidinyl-N-methylammonium)ethyl ester iodide, mp. 115-116° C;
naproxen 2-(4-morpholinyl-N-methylammonium)ethyl ester iodide, mp. 144-145° C; and
naproxen 2-(N,N-dibutyl-N-methylammonium)ethyl ester iodide, mp. 74-76° C.

(ii) From the ω-dialkylamino alcohols of formula (3) listed in Preparation 3.B:
naproxen 3-(N,N,N-trimethylammonium)propyl ester iodide, mp. 222-224° C;
naproxen 4-(N,N,N-trimethylammonium)butyl ester iodide, mp. 139-140° C;
naproxen 5-(N,N,N-trimethylammonium)pentyl ester idodide, mp. 105-106° C; and
naproxen 6-(N,N,N-trimethylammonium)hexyl ester iodide, mp. 79-81° C.

B.2.b. Similarly, by substituting other ω-dialkylamino alcohols of formula (3) listed in (i) Preparation 1.A. and B and (ii) Preparation 3.B above, for 2-dimethylaminoethanol, the following corresponding compounds of

Formula I are prepared in accordance with the procedure described in this Example 1, section A.0, above:

(i) From the ω-dialkylamino alcohols of formula (3) listed in Preparation 1.A and B:

naproxen 2-(N,N-dibenzyl-N-methylammonium)ethyl ester iodide;
naproxen 2-(N,N-diphenyl-N-methylammonium)ethyl ester iodide;
naproxen 2-(N,N-diethyl-N-methylammonium)ethyl ester iodide;
naproxen 2-(N,N-di-n-propyl-N-methylammonium)ethyl ester iodide;
naproxen 2-(N,N-di-n-butyl-N-methylammonium)ethyl ester iodide;
naproxen 2-(N,N-dimethyl-N-benzylammonium)ethyl ester iodide;
naproxen 2-(N-phenyl-N-benzyl-N-methylammonium)ethyl ester iodide;
naproxen 2-(N-methyl-N-ethyl-N-butylammonium)ethyl ester iodide; and
naproxen 2-(N-methyl-N-ethyl-N-phenylammonium)ethyl ester iodide.

(ii) From the ω-dialkylamino alcohols of formula (3) listed in Preparation 3.B:

naproxen 4-(4-morpholinyl-N-methylammonium)butyl ester iodide;
naproxen 4-(1-piperidinyl-N-methylammonium)butyl ester iodide;
naproxen 4-(1-pyrrolidinyl-N-methylammonium)butyl ester iodide;
naproxen 4-(N,N-diphenyl-N-methylammonium)butyl ester iodide;
naproxen 4-(N,N-diethyl-N-methylammonium)butyl ester iodide;
naproxen 4-(N,N-di-isopropyl-N-methylammonium)butyl ester iodide;
naproxen 4-(N,N-di-n-butyl-N-methylammonium)butyl ester iodide;
naproxen 4-(N,N-dimethyl-N-benzylammonium)butyl ester iodide;
naproxen 4-(N-phenyl-N-benzyl-N-methylammonium)butyl ester iodide;
naproxen 4-(N-methyl-N-ethyl-N-butylammonium)butyl ester iodide;
naproxen 4-(N-methyl-N-ethyl-N-phenylammonium)butyl ester iodide;
naproxen 3-(4-morpholinyl-N-methylammonium)propyl ester iodide;
naproxen 3-(1-piperidinyl-N-methylammonium)propyl ester iodide;
naproxen 3-(1-pyrrolidinyl-N-methylammonium)propyl ester iodide;
naproxen 3-(N,N-diphenyl-N-methylammonium)propyl ester iodide;
naproxen 3-(N,N-diethyl-N-methylammonium)propyl ester iodide;
naproxen 3-(N,N-di-isopropyl-N-methylammonium)propyl ester iodide;
naproxen 3-(N,N-di-n-butyl-N-methylammonium)propyl ester iodide;
naproxen 3-(N,N-dimethyl-N-benzylammonium)propyl ester iodide;
naproxen 3-(N-phenyl-N-benzyl-N-methylammonium)propyl ester iodide;
naproxen 3-(N-methyl-N-ethyl-N-butylammonium)propyl ester iodide;
naproxen 3-(N-methyl-N-ethyl-N-phenylammonium)propyl ester iodide;
naproxen 5-(4-morpholinyl-N-methylammonium)pentyl ester iodide;
naproxen 5-(1-piperidinyl-N-methylammonium)pentyl ester iodide;
naproxen 5-(1-pyrrolidinyl-N-methylammonium)pentyl ester iodide;
naproxen 5-(N,N,N-trimethylammonium)pentyl ester iodide, mp. 105-106°C;
naproxen 5-(N,N-diphenyl-N-methylammonium)pentyl ester iodide;
naproxen 5-(N,N-diethyl-N-methylammonium)pentyl ester iodide;
naproxen 5-(N,N-di-isopropyl-N-methylammonium)pentyl ester iodide;
naproxen 5-(N,N-di-n-butyl-N-methylammonium)pentyl ester iodide;
naproxen 5-(N,N-dimethyl-N-benzylammonium)pentyl ester iodide;
naproxen 5-(N-phenyl-N-benzyl-N-methylammonium)pentyl ester iodide;
naproxen 5-(N-methyl-N-ethyl-N-butylammonium)pentyl ester iodide;
naproxen 5-(N-methyl-N-ethyl-N-phenylammonium)pentyl ester iodide;
naproxen 6-(4-morpholinyl-N-methylammonium)hexyl ester iodide;
naproxen 6-(1-piperidinyl-N-methylammonium)hexyl ester iodide;
naproxen 6-(1-pyrrolidinyl-N-methylammonium)hexyl ester iodide;
naproxen 6-(N,N-diphenyl-N-methylammonium)hexyl ester iodide;
naproxen 6-(N,N-diethyl-N-methylammonium)hexyl ester iodide;
naproxen 6-(N,N-di-isopropyl-N-methylammonium)hexyl ester iodide;
naproxen 6-(N,N-di-n-butyl-N-methylammonium)hexyl ester iodide;
naproxen 6-(N,N-dimethyl-N-benzylammonium)hexyl ester iodide;
naproxen 6-(N-phenyl-N-benzyl-N-methylammonium)hexyl ester iodide;
naproxen 6-(N-methyl-N-ethyl-N-butylammonium)hexyl ester iodide;
naproxen 6-(N-methyl-N-ethyl-N-phenylammonium)hexyl ester iodide.

B.3.a. Similarly, by substituting 2-diethylaminoethanol for 2-dimethylaminoethanol and ethyl iodide for methyl iodide, the following compound of Formula I was prepared in accordance with the procedure described in this Example 1, section A.0, above:

naproxen 2-(N,N,N-triethylammonium)ethyl ester iodide, mp. 54-56°C.

B.3.b. Similarly, by optionally substituting other ω-dialkylamino alcohols for 2-dimethylaminoethanol of formula (3) listed in (i) Preparation 1 and (ii) Preparation 3 above, for 2-dimethylaminoethanol, and other alkylating agents $R^3X$ for methyl iodide, the following corresponding compounds of Formula I are prepared in accordance with the procedure described in this Example 1, section A.0, above:

(i) From the ω-dialkylamino alcohols of formula (3) listed in Preparation 1:
naproxen 3-(N,N,N-triethylammonium)ethyl ester chloride;
naproxen 2-(1-piperidinyl-N-methylammonium)ethyl ester chloride;
naproxen 2-(1-pyrrolidinyl-N-methylammonium)ethyl ester chloride;
naproxen 2-(4-morpholinyl-N-methylammonium)ethyl ester chloride;
naproxen 2-(N,N-dibutyl-N-methylammonium)ethyl ester chloride;
naproxen 2-(N,N-dibenzyl-N-methylammonium)ethyl ester chloride;
naproxen 2-(N,N-diphenyl-N-methylammonium)ethyl ester chloride;
naproxen 2-(N,N-diethyl-N-methylammonium)ethyl ester chloride;
naproxen 2-(N,N-di-n-propyl-N-methylammonium)ethyl ester chloride;
naproxen 2-(N,N-di-n-butyl-N-methylammonium)ethyl ester chloride;
naproxen 2-(N,N-dimethyl-N-benzylammonium)ethyl ester chloride;
naproxen 2-(N-phenyl-N-benzyl-N-methylammonium)ethyl ester chloride;
naproxen 2-(N-methyl-N-ethyl-N-butylammonium)ethyl ester chloride;
naproxen 2-(N-methyl-N-ethyl-N-phenylammonium)ethyl ester chloride;
naproxen 2-(N,N,N-trimethylammonium)ethyl ester bromide;
naproxen 2-(1-piperidinyl-N-methylammonium)ethyl ester bromide;
naproxen 2-(1-pyrrolidinyl-N-methylammonium)ethyl ester bromide;
naproxen 2-(4-morpholinyl-N-methylammonium)ethyl ester bromide;
naproxen 2-(N,N-dibutyl-N-methylammonium)ethyl ester bromide;
naproxen 2-(N,N-dibenzyl-N-methylammonium)ethyl ester bromide;
naproxen 2-(N,N-diphenyl-N-methylammonium)ethyl ester bromide;
naproxen 2-(N,N-diethyl-N-methylammonium)ethyl ester bromide;
naproxen 2-(N,N-di-n-propyl-N-methylammonium)ethyl ester bromide;
naproxen 2-(N,N-di-n-butyl-N-methylammonium)ethyl ester bromide;
naproxen 2-(N,N-dimethyl-N-benzylammonium)ethyl ester bromide;
naproxen 2-(N-phenyl-N-benzyl-N-methylammonium)ethyl ester bromide;
naproxen 2-(N-methyl-N-ethyl-N-butylammonium)ethyl ester bromide; and
naproxen 2-(N-methyl-N-ethyl-N-phenylammonium)ethyl ester bromide.

(ii) From the ω-dialkylamino alcohols of formula (3) listed in Preparation 3:
naproxen 3-(N,N,N-trimethylammonium)propyl ester chloride;
naproxen 4-(N,N,N-trimethylammonium)butyl ester chloride;
naproxen 6-(N,N,N-trimethylammonium)hexyl ester chloride;
naproxen 4-(4-morpholinyl-N-methylammonium)butyl ester chloride;
naproxen 4-(1-piperidinyl-N-methylammonium)butyl ester chloride;
naproxen 4-(1-pyrrolidinyl-N-methylammonium)butyl ester chloride;
naproxen 4-(N,N-diphenyl-N-methylammonium)butyl ester chloride;
naproxen 4-(N,N-diethyl-N-methylammonium)butyl ester chloride;
naproxen 4-(N,N-di-isopropyl-N-methylammonium)butyl ester chloride;
naproxen 4-(N,N-di-n-butyl-N-methylammonium)butyl ester chloride;
naproxen 4-(N,N-dimethyl-N-benzylammonium)butyl ester chloride;
naproxen 4-(N-phenyl-N-benzyl-N-methylammonium)butyl ester chloride;
naproxen 4-(N-methyl-N-ethyl-N-butylammonium)butyl ester chloride;
naproxen 4-(N-methyl-N-ethyl-N-phenylammonium)butyl ester chloride;
naproxen 3-(4-morpholinyl-N-methylammonium)propyl ester chloride;
naproxen 3-(1-piperidinyl-N-methylammonium)propyl ester chloride;
naproxen 3-(1-pyrrolidinyl-N-methylammonium)propyl ester chloride;
naproxen 3-(N,N-diphenyl-N-methylammonium)propyl ester chloride;
naproxen 3-(N,N-diethyl-N-methylammonium)propyl ester chloride;
naproxen 3-(N,N-di-isopropyl-N-methylammonium)propyl ester chloride;
naproxen 3-(N,N-di-n-butyl-N-methylammonium)propyl ester chloride;
naproxen 3-(N,N-dimethyl-N-benzylammonium)propyl ester chloride;
naproxen 3-(N-phenyl-N-benzyl-N-methylammonium)propyl ester chloride;
naproxen 3-(N-methyl-N-ethyl-N-butylammonium)propyl ester chloride;
naproxen 3-(N-methyl-N-ethyl-N-phenylammonium)propyl ester chloride;
naproxen 5-(4-morpholinyl-N-methylammonium)pentyl ester chloride;
naproxen 5-(1-piperidinyl-N-methylammonium)pentyl ester chloride;
naproxen 5-(1-pyrrolidinyl-N-methylammonium)pentyl ester chloride;
naproxen 5-(N,N,N-trimethylammonium)pentyl ester chloride;
naproxen 5-(N,N-diphenyl-N-methylammonium)pentyl ester chloride;
naproxen 5-(N,N-diethyl-N-methylammonium)pentyl ester chloride;
naproxen 5-(N,N-di-isopropyl-N-methylammonium)pentyl ester chloride;
naproxen 5-(N,N-di-n-butyl-N-methylammonium)pentyl ester chloride;
naproxen 5-(N,N-dimethyl-N-benzylammonium)pentyl ester chloride;
naproxen 5-(N-phenyl-N-benzyl-N-methylammonium)pentyl ester chloride;

naproxen 5-(N-methyl-N-ethyl-N-butylammonium)pentyl ester chloride;
naproxen 5-(N-methyl-N-ethyl-N-phenylammonium)pentyl ester chloride;
naproxen 6-(4-morpholinyl-N-methylammonium)hexyl ester chloride;
naproxen 6-(1-piperidinyl-N-methylammonium)hexyl ester chloride;
naproxen 6-(1-pyrrolidinyl-N-methylammonium)hexyl ester chloride;
naproxen 6-(N,N-diphenyl-N-methylammonium)hexyl ester chloride;
naproxen 6-(N,N-diethyl-N-methylammonium)hexyl ester chloride;
naproxen 6-(N,N-di-isopropyl-N-methylammonium)hexyl ester chloride;
naproxen 6-(N,N-di-n-butyl-N-methylammonium)hexyl ester chloride;
naproxen 6-(N,N-dimethyl-N-benzylammonium)hexyl ester chloride;
naproxen 6-(N-phenyl-N-benzyl-N-methylammonium)hexyl ester chloride;
naproxen 6-(N-methyl-N-ethyl-N-butylammonium)hexyl ester chloride;
naproxen 6-(N-methyl-N-ethyl-N-phenylammonium)hexyl ester chloride;
naproxen 3-(N,N,N-trimethylammonium)propyl ester bromide;
naproxen 4-(N,N,N-trimethylammonium)butyl ester bromide;
naproxen 6-(N,N,N-trimethylammonium)hexyl ester bromide;
naproxen 4-(4-morpholinyl-N-methylammonium)butyl ester bromide;
naproxen 4-(1-piperidinyl-N-methylammonium)butyl ester bromide;
naproxen 4-(1-pyrrolidinyl-N-methylammonium)butyl ester bromide;
naproxen 4-(N,N-diphenyl-N-methylammonium)butyl ester bromide;
naproxen 4-(N,N-diethyl-N-methylammonium)butyl ester bromide;
naproxen 4-(N,N-di-isopropyl-N-methylammonium)butyl ester bromide;
naproxen 4-(N,N-di-n-butyl-N-methylammonium)butyl ester bromide;
naproxen 4-(N,N-dimethyl-N-benzylammonium)butyl ester bromide;
naproxen 4-(N-phenyl-N-benzyl-N-methylammonium)butyl ester bromide;
naproxen 4-(N-methyl-N-ethyl-N-butylammonium)butyl ester bromide;
naproxen 4-(N-methyl-N-ethyl-N-phenylammonium)butyl ester bromide;
naproxen 3-(4-morpholinyl-N-methylammonium)propyl ester bromide;
naproxen 3-(1-piperidinyl-N-methylammonium)propyl ester bromide;
naproxen 3-(1-pyrrolidinyl-N-methylammonium)propyl ester bromide;
naproxen 3-(N,N-diphenyl-N-methylammonium)propyl ester bromide;
naproxen 3-(N,N-diethyl-N-methylammonium)propyl ester bromide;
naproxen 3-(N,N-di-isopropyl-N-methylammonium)propyl ester bromide;
naproxen 3-(N,N-di-n-butyl-N-methylammonium)propyl ester bromide;
naproxen 3-(N,N-dimethyl-N-benzylammonium)propyl ester bromide;
naproxen 3-(N-phenyl-N-benzyl-N-methylammonium)propyl ester bromide;
naproxen 3-(N-methyl-N-ethyl-N-butylammonium)propyl ester bromide;
naproxen 3-(N-methyl-N-ethyl-N-phenylammonium)propyl ester bromide;
naproxen 5-(4-morpholinyl-N-methylammonium)pentyl ester bromide;
naproxen 5-(1-piperidinyl-N-methylammonium)pentyl ester bromide;
naproxen 5-(1-pyrrolidinyl-N-methylammonium)pentyl ester bromide;
naproxen 5-(N,N,N-trimethylammonium)pentyl ester bromide;
naproxen 5-(N,N-diphenyl-N-methylammonium)pentyl ester bromide;
naproxen 5-(N,N-diethyl-N-methylammonium)pentyl ester bromide;
naproxen 5-(N,N-di-isopropyl-N-methylammonium)pentyl ester bromide;
naproxen 5-(N,N-di-n-butyl-N-methylammonium)pentyl ester bromide;
naproxen 5-(N,N-dimethyl-N-benzylammonium)pentyl ester bromide;
naproxen 5-(N-phenyl-N-benzyl-N-methylammonium)pentyl ester bromide;
naproxen 5-(N-methyl-N-ethyl-N-butylammonium)pentyl ester bromide;
naproxen 5-(N-methyl-N-ethyl-N-phenylammonium)pentyl ester bromide;
naproxen 6-(4-morpholinyl-N-methylammonium)hexyl ester bromide;
naproxen 6-(1-piperidinyl-N-methylammonium)hexyl ester bromide;
naproxen 6-(1-pyrrolidinyl-N-methylammonium)hexyl ester bromide;
naproxen 6-(N,N-diphenyl-N-methylammonium)hexyl ester bromide;
naproxen 6-(N,N-diethyl-N-methylammonium)hexyl ester bromide;
naproxen 6-(N,N-di-isopropyl-N-methylammonium)hexyl ester bromide;
naproxen 6-(N,N-di-n-butyl-N-methylammonium)hexyl ester bromide;
naproxen 6-(N,N-dimethyl-N-benzylammonium)hexyl ester bromide;
naproxen 6-(N-phenyl-N-benzyl-N-methylammonium)hexyl ester bromide;
naproxen 6-(N-methyl-N-ethyl-N-butylammonium)hexyl ester bromide; and
naproxen 6-(N-methyl-N-ethyl-N-phenylammonium)hexyl ester bromide.

B.4. Similarly, by substituting: optionally other ω-dialkylamino alcohols (such as those listed, for example, in Preparations 1 and 3) for 2-dimethylaminoethanol; other ANSAIDs (such as those listed, for example, in Example 1 A.1 and A.2) for naproxen; and optionally other alkylating agents $R^3X$ (such as ethyl iodide, methyl bromide, methyl chloride, and the like) for methyl iodide, the following compounds of Formula I are prepared in

accordance with the procedure described in this Example 1, section A.0, above:

(i) From the ω-dialkylamino alcohols of formula (3) listed in Preparation 1:

ketorolac 3-(N,N,N-triethylammonium)ethyl ester chloride;
ketorolac 4-(N,N,N-trimethylammonium)butyl ester idodie, mp. 138-140° C;
ketorolac 2-(1-piperidinyl-N-methylammonium)ethyl ester chloride;
ketorolac 2-(1-pyrrolidinyl-N-methylammonium)ethyl ester chloride;
ketorolac 2-(4-morpholinyl-N-methylammonium)ethyl ester chloride;
ketorolac 2-(N,N-dibutyl-N-methylammonium)ethyl ester chloride;
ketorolac 2-(N,N-dibenzyl-N-methylammonium)ethyl ester chloride;
indomethacin 2-(N,N-diphenyl-N-methylammonium)ethyl ester chloride;
indomethacin 2-(N,N-diethyl-N-methylammonium)ethyl ester chloride;
indomethacin 2-(N,N-di-n-propyl-N-methylammonium)ethyl ester chloride;
indomethacin 2-(N,N-di-n-butyl-N-methylammonium)ethyl ester chloride;
indomethacin 2-(N,N-dimethyl-N-benzylammonium)ethyl ester chloride;
indomethacin 2-(N-phenyl-N-benzyl-N-methylammonium)ethyl ester chloride;
sulindac 2-(N-methyl-N-ethyl-N-butylammonium)ethyl ester chloride;
sulindac 2-(N-methyl-N-ethyl-N-phenylammonium)ethyl ester chloride;
sulindac 2-(N,N,N-trimethylammonium)ethyl ester bromide;
sulindac 2-(1-piperidinyl-N-methylammonium)ethyl ester bromide;
sulindac 2-(1-pyrrolidinyl-N-methylammonium)ethyl ester bromide;
sulindac 2-(4-morpholinyl-N-methylammonium)ethyl ester bromide;
ibuprofen 2-(N,N-dibutyl-N-methylammonium)ethyl ester bromide;
ibuprofen 2-(N,N-dibenzyl-N-methylammonium)ethyl ester bromide;
ibuprofen 2-(N,N-diphenyl-N-methylammonium)ethyl ester bromide;
ibuprofen 2-(N,N-diethyl-N-methylammonium)ethyl ester bromide;
ibuprofen 2-(N,N-di-n-propyl-N-methylammonium)ethyl ester bromide;
ibuprofen 2-(N,N-di-n-butyl-N-methylammonium)ethyl ester bromide;
zomepirac 2-(N,N-dimethyl-N-benzylammonium)ethyl ester bromide;
zomepirac 2-(N-phenyl-N-benzyl-N-methylammonium)ethyl ester bromide;
zomepirac 2-(N-methyl-N-ethyl-N-butylammonium)ethyl ester bromide; and
zomepirac 2-(N-methyl-N-ethyl-N-phenylammonium)ethyl ester bromide.

(ii) From the ω-dialkylamino alcohols of formula (3) listed in Preparation 3:

zomepirac 3-(N,N,N-trimethylammonium)propyl ester chloride;
zomepirac 4-(N,N,N-trimethylammonium)butyl ester chloride;
flufenamic acid 6-(N,N,N-trimethylammonium)hexyl ester chloride;
anirolac 4-(4-morpholinyl-N-methylammonium)butyl ester chloride;
ketorolac 4-(1-piperidinyl-N-methylammonium)butyl ester chloride;
tifurac 4-(1-pyrrolidinyl-N-methylammonium)butyl ester chloride;
flufenamic acid 4-(N,N-diphenyl-N-methylammonium)butyl ester chloride;
flufenamic acid 4-(N,N-diethyl-N-methylammonium)butyl ester chloride;
flufenamic acid 4-(N,N-di-isopropyl-N-methylammonium)butyl ester chloride;
flufenamic acid 4-(N,N-di-n-butyl-N-methylammonium)butyl ester chloride;
flufenamic acid 4-(N,N-dimethyl-N-benzylammonium)butyl ester chloride;
ketoprofen 4-(N-phenyl-N-benzyl-N-methylammonium)butyl ester chloride;
ketoprofen 4-(N-methyl-N-ethyl-N-butylammonium)butyl ester chloride;
ketoprofen 4-(N-methyl-N-ethyl-N-phenylammonium)butyl ester chloride;
anirolac 3-(4-morpholinyl-N-methylammonium)propyl ester chloride;
ketorolac 3-(1-piperidinyl-N-methylammonium)propyl ester chloride;
tifurac 3-(1-pyrrolidinyl-N-methylammonium)propyl ester chloride;
ketoprofen 3-(N,N-diphenyl-N-methylammonium)propyl ester chloride;
ketoprofen 3-(N,N-diethyl-N-methylammonium)propyl ester chloride;
ketoprofen 3-(N,N-di-isopropyl-N-methylammonium)propyl ester chloride;
benoxaprofen 3-(N,N-di-n-butyl-N-methylammonium)propyl ester chloride;
benoxaprofen 3-(N,N-dimethyl-N-benzylammonium)propyl ester chloride;
benoxaprofen 3-(N-phenyl-N-benzyl-N-methylammonium)propyl ester chloride;
benoxaprofen 3-(N-methyl-N-ethyl-N-butylammonium)propyl ester chloride;
benoxaprofen 3-(N-methyl-N-ethyl-N-phenylammonium)propyl ester chloride;
anirolac 5-(4-morpholinyl-N-methylammonium)pentyl ester chloride;
ketorolac 5-(1-piperidinyl-N-methylammonium)pentyl ester chloride;
tifurac 5-(1-pyrrolidinyl-N-methylammonium)pentyl ester chloride;
benoxaprofen 5-(N,N,N-trimethylammonium)pentyl ester chloride;
etodolac 5-(N,N-diphenyl-N-methylammonium)pentyl ester chloride;
etodolac 5-(N,N-diethyl-N-methylammonium)pentyl ester chloride;
etodolac 5-(N,N-di-isopropyl-N-methylammonium)pentyl ester chloride;
etodolac 5-(N,N-di-n-butyl-N-methylammonium)pentyl ester chloride;

etodolac 5-(N,N-dimethyl-N-benzylammonium)pentyl ester chloride;
etodolac 5-(N-phenyl-N-benzyl-N-methylammonium)pentyl ester chloride;
tolmetin 5-(N-methyl-N-ethyl-N-butylammonium)pentyl ester chloride;
tolmetin 5-(N-methyl-N-ethyl-N-phenylammonium)pentyl ester chloride;
anirolac 6-(4-morpholinyl-N-methylammonium)hexyl ester chloride;
ketorolac 6-(1-piperidinyl-N-methylammonium)hexyl ester chloride;
tifurac 6-(1-pyrrolidinyl-N-methylammonium)hexyl ester chloride;
tolmetin 6-(N,N-diphenyl-N-methylammonium)hexyl ester chloride;
tolmetin 6-(N,N-diethyl-N-methylammonium)hexyl ester chloride;
tolmetin 6-(N,N-di-isopropyl-N-methylammonium)hexyl ester chloride;
tolmetin 6-(N,N-di-n-butyl-N-methylammonium)hexyl ester chloride;
tifurac 6-(N,N-dimethyl-N-benzylammonium)hexyl ester chloride;
tifurac 6-(N-phenyl-N-benzyl-N-methylammonium)hexyl ester chloride;
tifurac 6-(N-methyl-N-ethyl-N-butylammonium)hexyl ester chloride;
tifurac 6-(N-methyl-N-ethyl-N-phenylammonium)hexyl ester chloride;
tifurac 3-(N,N,N-trimethylammonium)propyl ester bromide;
tifurac 4-(N,N,N-trimethylammonium)butyl ester bromide;
diclofenac 6-(N,N,N-trimethylammonium)hexyl ester bromide;
anirolac 4-(4-morpholinyl-N-methylammonium)butyl ester bromide;
ketorolac 4-(1-piperidinyl-N-methylammonium)butyl ester bromide;
tifurac 4-(1-pyrrolidinyl-N-methylammonium)butyl ester bromide;
diclofenac 4-(N,N-diphenyl-N-methylammonium)butyl ester bromide;
diclofenac 4-(N,N-diethyl-N-methylammonium)butyl ester bromide;
diclofenac 4-(N,N-di-isopropyl-N-methylammonium)butyl ester bromide;
diclofenac 4-(N,N-di-n-butyl-N-methylammonium)butyl ester bromide;
diclofenac 4-(N,N-dimethyl-N-benzylammonium)butyl ester bromide;
fenclofenac 4-(N-phenyl-N-benzyl-N-methylammonium)butyl ester bromide;
fenclofenac 4-(N-methyl-N-ethyl-N-butylammonium)butyl ester bromide;
fenclofenac 4-(N-methyl-N-ethyl-N-phenylammonium)butyl ester bromide;
anirolac 3-(4-morpholinyl-N-methylammonium)propyl ester bromide;
ketorolac 3-(1-piperidinyl-N-methylammonium)propyl ester bromide;
tifurac 3-(1-pyrrolidinyl-N-methylammonium)propyl ester bromide;
fenclofenac 3-(N,N-diphenyl-N-methylammonium)propyl ester bromide;
fenclofenac 3-(N,N-diethyl-N-methylammonium)propyl ester bromide;
fenclofenac 3-(N,N-di-isopropyl-N-methylammonium)propyl ester bromide;
anirolac 3-(N,N-di-n-butyl-N-methylammonium)propyl ester bromide;
anirolac 3-(N,N-dimethyl-N-benzylammonium)propyl ester bromide;
anirolac 3-(N-phenyl-N-benzyl-N-methylammonium)propyl ester bromide;
anirolac 3-(N-methyl-N-ethyl-N-butylammonium)propyl ester bromide;
anirolac 3-(N-methyl-N-ethyl-N-phenylammonium)propyl ester bromide;
anirolac 5-(4-morpholinyl-N-methylammonium)pentyl ester bromide;
ketorolac 5-(1-piperidinyl-N-methylammonium)pentyl ester bromide;
tifurac 5-(1-pyrrolidinyl-N-methylammonium)pentyl ester bromide;
anirolac 5-(N,N,N-trimethylammonium)pentyl ester bromide;
mefenamic acid 5-(N,N-diphenyl-N-methylammonium)pentyl ester bromide;
mefenamic acid 5-(N,N-diethyl-N-methylammonium)pentyl ester bromide;
mefenamic acid 5-(N,N-di-isopropyl-N-methylammonium)pentyl ester bromide;
mefenamic acid 5-(N,N-di-n-butyl-N-methylammonium)pentyl ester bromide;
mefenamic acid 5-(N,N-dimethyl-N-benzylammonium)pentyl ester bromide;
mefenamic acid 5-(N-phenyl-N-benzyl-N-methylammonium)pentyl ester bromide;
ketorolac 5-(N-methyl-N-ethyl-N-butylammonium)pentyl ester bromide;
ketorolac 5-(N-methyl-N-ethyl-N-phenylammonium)pentyl ester bromide;
anirolac 6-(4-morpholinyl-N-methylammonium)hexyl ester bromide;
ketorolac 6-(1-piperidinyl-N-methylammonium)hexyl ester bromide;
tifurac 6-(1-pyrrolidinyl-N-methylammonium)hexyl ester bromide;
ketorolac 6-(N,N-diphenyl-N-methylammonium)hexyl ester bromide;
ketorolac 6-(N,N-diethyl-N-methylammonium)hexyl ester bromide;
anirolac 6-(N,N-di-isopropyl-N-methylammonium)hexyl ester bromide;
anirolac 6-(N,N-di-n-butyl-N-methylammonium)hexyl ester bromide;
anirolac 6-(N,N-dimethyl-N-benzylammonium)hexyl ester bromide;
anirolac 6-(N-phenyl-N-benzyl-N-methylammonium)hexyl ester bromide;
tifurac 6-(N-methyl-N-ethyl-N-butylammonium)hexyl ester bromide; and
tifurac 6-(N-methyl-N-ethyl-N-phenylammonium)hexyl ester bromide.

It will be apparent to one of ordinary skill in the art that the above listing is for the purpose of exemplification, and is not exhaustive. Accordingly, any of the ANSAIDs listed in parts A.1 and 2 of this Example 1 can be used

in place of naproxen to obtain the analogous esters to the naproxen esters listed in parts B.2 and B.3 of this Example 1. Moreover, other ANSAIDS (RCOOH) can be substituted, as well as other dialkylaminoalcohols of formula (3), as well as other alkylating agents $R^3X$.

EXAMPLE 2

NAPROXEN THIOCHOLINE ESTER IODIDE

A.0 A solution of naproxen (11.51 g, 50 mmol) in dry tetrahydrofuran (100 ml) was treated with solid 1,1′-carbonyldiimidazole (9.3g), and was stirred 1 hour at room temperature. To this solution was added a tetrahydrofuran solution prepared from 2-dimethylaminoethanethiol (10.6 g) and n-butyllithium (53 ml of 1.5 M solution in hexane), and the resulting mixture was stirred at room temperature overnight. The solvent was removed by evaporation, and the residue was dissolved in diethyl ether and washed with water and brine. The organic extract was dried over sodium sulfate, and evaporated to afford an oil. The oily ester was dissolved in fresh diethyl ether, and was treated with methyl iodide (9 ml). After stirring at ambient temperature overnight, the resulting precipitate llected by filtration, washed with additional l ether and dried in vacuo to afford the title compound (22.0 g, 47.8 mmol, 96%) as a white crystalline solid, mp 125-126° C.

A.1. Similarly, by substituting ketorolac, indomethacin, sulindac, ibuprofen, zomepirac, tifurac, flufenamic acid and ketoprofen, respectively, for naproxen, in the above section A.0 of this Example 2, the following compounds of Formula I were prepared:

ketorolac thiocholine ester iodide, mp. 118-120° C;
indomethacin thiocholine ester iodide, mp. 198-200° C;
sulindac thiocholine ester iodide, mp. 148-150° C;
ibuprofen thiocholine ester iodide, mp. 143-144° C;
zomepirac thiocholine ester iodide, mp. 161-162° C;
tifurac thiocholine ester iodide, mp. 146-147° C;
mefenamic acid thiocholine ester iodide, mp. 215-216° C;
flufenamic acid thiocholine ester iodide, mp. 100-101° C; and
ketoprofen thiocholine ester iodide, mp. 126-128° C.

A.2. Similarly, by substituting benoxaprofen, etodolac, tolmetin, diclofenac, fenclofenac, and anirolac, respectively, for naproxen, in the above section A.0 of this Example 2, the following compounds of Formula I are prepared:

benoxaprofen thiocholine ester iodide;
etodolac thiocholine ester iodide, mp. 126-128° C;
tolmetin thiocholine ester iodide;
diclofenac thiocholine iodide;
fenclofenac thiocholine ester iodide; and
anirolac thiocholine ester iodide.

B.1. Similarly, by optionally substituting other ANSAIDs (e.g., benoxaprofen, etodolac, tolmetin, etc. respectively - see A.1 and A.2 above) for naproxen and substituting other alkylating agents $R^3X$ for methyl iodide in the above section A.0 of this Example 2, the following compounds of Formula I are prepared:

ketorolac thiocholine ester chloride;
indomethacin thiocholine ester chloride;
sulindac thiocholine ester chloride;
ibuprofen thiocholine ester chloride;
zomepirac thiocholine ester chloride;
tifurac thiocholine ester chloride;
mefenamic acid thiocholine ester chloride;
flufenamic acid thiocholine ester chloride;
ketoprofen thiocholine ester chloride;
benoxaprofen thiocholine ester chloride;
etodolac thiocholine ester chloride;
tolmetin thiocholine ester chloride;
diclofenac thiocholine ester chloride;
fenclofenac thiocholine ester chloride;
anirolac thiocholine ester chloride;
ketorolac thiocholine ester bromide;
indomethacin thiocholine ester bromide;
sulindac thiocholine ester bromide;
ibuprofen thiocholine ester bromide;
zomepirac thiocholine ester bromide;
tifurac thiocholine ester bromide;
mefenamic acid thiocholine ester bromide;
flufenamic acid thiocholine ester bromide;
ketoprofen thiocholine ester bromide;
benoxaprofen thiocholine ester bromide;

etodolac thiocholine ester bromide;
tolmetin thiocholine ester bromide;
diclofenac thiocholine ester bromide;
fenclofenac thiocholine ester bromide; and
anirolac thiocholine ester bromide;

B.2. Similarly, by substituting other ω-dialkylamino alkyl thiols of formula (3) listed in (i) Preparation 2 and (ii) Preparation 4 above, for 2-dimethylaminoethanethiol, the following corresponding compounds of Formula I are prepared in accordance with the procedure described in this Example 2, section A.0, above:

(i) From the ω-dialkylamino alkylthiols of formula (3) listed in Preparation 2:

naproxen 3-(N,N,N-triethylammonium)thioethyl ester iodide;
naproxen 2-(1-piperidinyl-N-methylammonium)thioethyl ester iodide;
naproxen 2-(1-pyrrolidinyl-N-methylammonium)thioethyl ester iodide;
naproxen 2-(4-morpholinyl-N-methylammonium)thioethyl ester iodide;
naproxen 2-(N,N-dibutyl-N-methylammonium)thioethyl ester iodide;
naproxen 2-(N,N-dibenzyl-N-methylammonium)thioethyl ester iodide;
naproxen 2-(N,N-diphenyl-N-methylammonium)thioethyl ester iodide;
naproxen 2-(N,N-diethyl-N-methylammonium)thioethyl ester iodide;
naproxen 2-(N,N-di-n-propyl-N-methylammonium)thioethyl ester iodide;
naproxen 2-(N,N-di-n-butyl-N-methylammonium)thioethyl ester iodide;
naproxen 2-(N,N-dimethyl-N-benzylammonium)thioethyl ester iodide;
naproxen 2-(N-phenyl-N-benzyl-N-methylammonium)thioethyl ester iodide;
naproxen 2-(N-methyl-N-ethyl-N-butylammonium)thioethyl ester iodide; and
naproxen 2-(N-methyl-N-ethyl-N-phenylammonium)thioethyl ester iodide

(ii) From the ω-dialkylamino alkylthiols of formula (3) listed in Preparation 4:

naproxen 3-(N,N,N-trimethylammonium)thiopropyl ester iodide;
naproxen 4-(N,N,N-trimethylammonium)thiobutyl ester iodide;
naproxen 6-(N,N,N-trimethylammonium)thiohexyl ester iodide;
naproxen 4-(4-morpholinyl-N-methylammonium)thiobutyl ester iodide;
naproxen 4-(1-piperidinyl-N-methylammonium)thiobutyl ester iodide;
naproxen 4-(1-pyrrolidinyl-N-methylammonium)thiobutyl ester iodide;
naproxen 4-(N,N-diphenyl-N-methylammonium)thiobutyl ester iodide;
naproxen 4-(N,N-diethyl-N-methylammonium)thiobutyl ester iodide;
naproxen 4-(N,N-di-isopropyl-N-methylammonium)thiobutyl ester iodide;
naproxen 4-(N,N-di-n-butyl-N-methylammonium)thiobutyl ester iodide;
naproxen 4-(N,N-dimethyl-N-benzylammonium)thiobutyl ester iodide;
naproxen 4-(N-phenyl-N-benzyl-N-methylammonium)thiobutyl ester iodide;
naproxen 4-(N-methyl-N-ethyl-N-butylammonium)thiobutyl ester iodide;
naproxen 4-(N-methyl-N-ethyl-N-phenylammonium)thiobutyl ester iodide;
naproxen 3-(4-morpholinyl-N-methylammonium)thiopropyl ester iodide;
naproxen 3-(1-piperidinyl-N-methylammonium)thiopropyl ester iodide;
naproxen 3-(1-pyrrolidinyl-N-methylammonium)thiopropyl ester iodide;
naproxen 3-(N,N-diphenyl-N-methylammonium)thiopropyl ester iodide;
naproxen 3-(N,N-diethyl-N-methylammonium)thiopropyl ester iodide;
naproxen 3-(N,N-di-isopropyl-N-methylammonium)thiopropyl ester iodide;
naproxen 3-(N,N-di-n-butyl-N-methylammonium)thiopropyl ester iodide;
naproxen 3-(N,N-dimethyl-N-benzylammonium)thiopropyl ester iodide;
naproxen 3-(N-phenyl-N-benzyl-N-methylammonium)thiopropyl ester iodide;
naproxen 3-(N-methyl-N-ethyl-N-butylammonium)thiopropyl ester iodide;
naproxen 3-(N-methyl-N-ethyl-N-phenylammonium)thiopropyl ester iodide;
naproxen 5-(4-morpholinyl-N-methylammonium)thiopentyl ester iodide;
naproxen 5-(1-piperidinyl-N-methylammonium)thiopentyl ester iodide;
naproxen 5-(1-pyrrolidinyl-N-methylammonium)thiopentyl ester iodide;
naproxen 5-(N,N,N-trimethylammonium)thiopentyl ester iodide;
naproxen 5-(N,N-diphenyl-N-methylammonium)thiopentyl ester iodide;
naproxen 5-(N,N-diethyl-N-methylammonium)thiopentyl ester iodide;
naproxen 5-(N,N-di-isopropyl-N-methylammonium)thiopentyl ester iodide;
naproxen 5-(N,N-di-n-butyl-N-methylammonium)thiopentyl ester iodide;
naproxen 5-(N,N-dimethyl-N-benzylammonium)thiopentyl ester iodide;
naproxen 5-(N-phenyl-N-benzyl-N-methylammonium)thiopentyl ester iodide;
naproxen 5-(N-methyl-N-ethyl-N-butylammonium)thiopentyl ester iodide;
naproxen 5-(N-methyl-N-ethyl-N-phenylammonium)thiopentyl ester iodide;
naproxen 6-(4-morpholinyl-N-methylammonium)thiohexyl ester iodide;
naproxen 6-(1-piperidinyl-N-methylammonium)thiohexyl ester iodide;
naproxen 6-(1-pyrrolidinyl-N-methylammonium)thiohexyl ester iodide;
naproxen 6-(N,N-diphenyl-N-methylammonium)thiohexyl ester iodide

naproxen 6-(N,N-diethyl-N-methylammonium)thiohexyl ester iodide;
naproxen 6-(N,N-di-isopropyl-N-methylammonium)thiohexyl ester iodide;
naproxen 6-(N,N-di-n-butyl-N-methylammonium)thiohexyl ester iodide;
naproxen 6-(N,N-dimethyl-N-benzylammonium)thiohexyl ester iodide;
naproxen 6-(N-phenyl-N-benzyl-N-methylammonium)thiohexyl ester iodide;
naproxen 6-(N-methyl-N-ethyl-N-butylammonium)thiohexyl ester iodide; and
naproxen 6-(N-methyl-N-ethyl-N-phenylammonium)thiohexyl ester iodide.

B.3. Similarly, by optionally substituting other ω-dialkylamino alkylthiols for 2-dimethylaminoethanethiol of formula (3) listed in (i) Preparation 2 and (ii) Preparation 4 above, for 2-dimethylaminoethanethiol, and other alkylating agents $R^3X$ for methyl iodide, the following corresponding compounds of Formula I are prepared in accordance with the procedure described in this Example 2, section A.0, above:

(i) From the ω-dialkylamino alkylthiols of formula (3) listed in Preparation 2:
naproxen 3-(N,N,N-triethylammonium)thioethyl ester chloride;
naproxen 2-(1-piperidinyl-N-methylammonium)thioethyl ester chloride;
naproxen 2-(1-pyrrolidinyl-N-methylammonium)thioethyl ester chloride;
naproxen 2-(4-morpholinyl-N-methylammonium)thioethyl ester chloride;
naproxen 2-(N,N-dibutyl-N-methylammonium)thioethyl ester chloride;
naproxen 2-(N,N-dibenzyl-N-methylammonium)thioethyl ester chloride;
naproxen 2-(N,N-diphenyl-N-methylammonium)thioethyl ester chloride;
naproxen 2-(N,N-diethyl-N-methylammonium)thioethyl ester chloride;
naproxen 2-(N,N-di-n-propyl-N-methylammonium)thioethyl ester chloride;
naproxen 2-(N,N-di-n-butyl-N-methylammonium)thioethyl ester chloride;
naproxen 2-(N,N-dimethyl-N-benzylammonium)thioethyl ester chloride;
naproxen 2-(N-phenyl-N-benzyl-N-methylammonium)thioethyl ester chloride;
naproxen 2-(N-methyl-N-ethyl-N-butylammonium)thioethyl ester chloride;
naproxen 2-(N-methyl-N-ethyl-N-phenylammonium)thioethyl ester chloride
naproxen 2-(N,N,N-triethylammonium)thioethyl ester bromide;
naproxen 2-(1-piperidinyl-N-methylammonium)thioethyl ester bromide;
naproxen 2-(1-pyrrolidinyl-N-methylammonium)thioethyl ester bromide;
naproxen 2-(4-morpholinyl-N-methylammonium)thioethyl ester bromide;
naproxen 2-(N,N-dibutyl-N-methylammonium)thioethyl ester bromide;
naproxen 2-(N,N-dibenzyl-N-methylammonium)thioethyl ester bromide;
naproxen 2-(N,N-diphenyl-N-methylammonium)thioethyl ester bromide;
naproxen 2-(N,N-diethyl-N-methylammonium)thioethyl ester bromide;
naproxen 2-(N,N-di-n-propyl-N-methylammonium)thioethyl ester bromide;
naproxen 2-(N,N-di-n-butyl-N-methylammonium)thioethyl ester bromide;
naproxen 2-(N,N-dimethyl-N-benzylammonium)thioethyl ester bromide;
naproxen 2-(N-phenyl-N-benzyl-N-methylammonium)thioethyl ester bromide;
naproxen 2-(N-methyl-N-ethyl-N-butylammonium)thioethyl ester bromide; and
naproxen 2-(N-methyl-N-ethyl-N-phenylammonium)thioethyl ester bromide.

(ii) From the ω-dialkylamino alkylthiols of formula (3) listed in Preparation 4:
naproxen 3-(N,N,N-trimethylammonium)thiopropylester chloride;
naproxen 4-(N,N,N-trimethylammonium)thiobutylester chloride;
naproxen 6-(N,N,N-trimethylammonium)thiohexylester chloride;
naproxen 4-(4-morpholinyl-N-methylammonium)thiobutyl ester chloride;
naproxen 4-(1-piperidinyl-N-methylammonium)thiobutyl ester chloride;
naproxen 4-(1-pyrrolidinyl-N-methylammonium)thiobutyl ester chloride;
naproxen 4-(N,N-diphenyl-N-methylammonium)thiobutylester chloride;
naproxen 4-(N,N-diethyl-N-methylammonium)thiobutylester chloride;
naproxen 4-(N,N-di-isopropyl-N-methylammonium)thiobutylester chloride;
naproxen 4-(N,N-di-n-butyl-N-methylammonium)thiobutylester chloride;
naproxen 4-(N,N-dimethyl-N-benzylammonium)thiobutylester chloride;
naproxen 4-(N-phenyl-N-benzyl-N-methylammonium)thiobutylester chloride;
naproxen 4-(N-methyl-N-ethyl-N-butylammonium)thiobutyl ester chloride;
naproxen 4-(N-methyl-N-ethyl-N-phenylammonium)thiobutyl ester chloride;
naproxen 3-(4-morpholinyl-N-methylammonium)thiopropyl ester chloride;
naproxen 3-(1-piperidinyl-N-methylammonium)thiopropyl ester chloride;
naproxen 3-(1-pyrrolidinyl-N-methylammonium)thiopropyl ester chloride;
naproxen 3-(N,N-diphenyl-N-methylammonium)thiopropyl ester chloride;
naproxen 3-(N,N-diethyl-N-methylammonium)thiopropyl ester chloride;
naproxen 3-(N,N-di-isopropyl-N-methylammonium)thiopropyl ester chloride;
naproxen 3-(N,N-di-n-butyl-N-methylammonium)thiopropyl ester chloride;
naproxen 3-(N,N-dimethyl-N-benzylammonium)thiopropyl ester chloride;
naproxen 3-(N-phenyl-N-benzyl-N-methylammonium)thiopropyl ester chloride;
naproxen 3-(N-methyl-N-ethyl-N-butylammonium)thiopropyl ester chloride;

naproxen 3-(N-methyl-N-ethyl-N-phenylammonium)thiopropyl ester chloride;
naproxen 5-(4-morpholinyl-N-methylammonium)thiopentyl ester chloride;
naproxen 5-(1-piperidinyl-N-methylammonium)thiopentyl ester chloride;
naproxen 5-(1-pyrrolidinyl-N-methylammonium)thiopentyl ester chloride;
naproxen 5-(N,N,N-trimethylammonium)thiopentyl ester chloride;
naproxen 5-(N,N-diphenyl-N-methylammonium)thiopentyl ester chloride;
naproxen 5-(N,N-diethyl-N-methylammonium)thiopentyl ester chloride;
naproxen 5-(N,N-di-isopropyl-N-methylammonium)thio pentyl ester chloride;
naproxen 5-(N,N-di-n-butyl-N-methylammonium)thiopentyl ester chloride;
naproxen 5-(N,N-dimethyl-N-benzylammonium)thiopentyl ester chloride;
naproxen 5-(N-phenyl-N-benzyl-N-methylammonium)thiopentyl ester chloride;
naproxen 5-(N-methyl-N-ethyl-N-butylammonium)thiopentyl ester chloride;
naproxen 5-(N-methyl-N-ethyl-N-phenylammonium)thiopentyl ester chloride;
naproxen 6-(4-morpholinyl-N-methylammonium)thiohexyl ester chloride;
naproxen 6-(1-piperidinyl-N-methylammonium)thiohexyl ester chloride;
naproxen 6-(1-pyrrolidinyl-N-methylammonium)thiohexyl ester chloride;
naproxen 6-(N,N-diphenyl-N-methylammonium)thiohexyl ester chloride;
naproxen 6-(N,N-diethyl-N-methylammonium)thiohexyl ester chloride;
naproxen 6-(N,N-di-isopropyl-N-methylammonium)thiohexyl ester chloride;
naproxen 6-(N,N-di-n-butyl-N-methylammonium)thiohexyl ester chloride;
naproxen 6-(N,N-dimethyl-N-benzylammonium)thiohexyl ester chloride;
naproxen 6-(N-phenyl-N-benzyl-N-methylammonium)thiohexyl ester chloride;
naproxen 6-(N-methyl-N-ethyl-N-butylammonium)thiohexyl ester chloride;
naproxen 6-(N-methyl-N-ethyl-N-phenylammonium)thiohexyl ester chloride;
naproxen 3-(N,N,N-trimethylammonium)thiopropyl ester bromide;
naproxen 4-(N,N,N-trimethylammonium)thiobutyl ester bromide;
naproxen 6-(N,N,N-trimethylammonium)thiohexyl ester bromide;
naproxen 4-(4-morpholinyl-N-methylammonium)thiobutyl ester bromide;
naproxen 4-(1-piperidinyl-N-methylammonium)thiobutyl ester bromide;
naproxen 4-(1-pyrrolidinyl-N-methylammonium)thiobutyl ester bromide;
naproxen 4-(N,N-diphenyl-N-methylammonium)thiobutylester bromide;
naproxen 4-(N,N-diethyl-N-methylammonium)thiobutylester bromide;
naproxen 4-(N,N-di-isopropyl-N-methylammonium)thiobutyl ester bromide;
naproxen 4-(N,N-di-n-butyl-N-methylammonium)thiobutyl ester bromide;
naproxen 4-(N,N-dimethyl-N-benzylammonium)thiobutylester bromide;
naproxen 4-(N-phenyl-N-benzyl-N-methylammonium)thiobutyl ester bromide;
naproxen 4-(N-methyl-N-ethyl-N-butylammonium)thiobutyl ester bromide;
naproxen 4-(N-methyl-N-ethyl-N-phenylammonium)thiobutyl ester bromide;
naproxen 3-(4-morpholinyl-N-methylammonium)thiopropyl ester chloride;
naproxen 3-(1-piperidinyl-N-methylammonium)thiopropyl ester bromide;
naproxen 3-(1-pyrrolidinyl-N-methylammonium)thiopropyl ester bromide;
naproxen 3-(N,N-diphenyl-N-methylammonium)thiopropyl ester bromide;
naproxen 3-(N,N-diethyl-N-methylammonium)thiopropyl ester bromide;
naproxen 3-(N,N-di-isopropyl-N-methylammonium)thiopropyl ester bromide;
naproxen 3-(N,N-di-n-butyl-N-methylammonium)thiopropyl ester bromide;
naproxen 3-(N,N-dimethyl-N-benzylammonium)thiopropyl ester bromide;
naproxen 3-(N-phenyl-N-benzyl-N-methylammonium)thiopropyl ester bromide;
naproxen 3-(N-methyl-N-ethyl-N-butylammonium)thiopropyl ester bromide;
naproxen 3-(N-methyl-N-ethyl-N-phenylammonium)thiopropyl ester bromide;
naproxen 5-(4-morpholinyl-N-methylammonium)thiopentyl ester bromide;
naproxen 5-(1-piperidinyl-N-methylammonium)thiopentyl ester bromide;
naproxen 5-(1-pyrrolidinyl-N-methylammonium)thiopentyl ester bromide;
naproxen 5-(N,N,N-trimethylammonium)thiopentyl ester bromide;
naproxen 5-(N,N-diphenyl-N-methylammonium)thiopentyl ester bromide;
naproxen 5-(N,N-diethyl-N-methylammonium)thiopentyl ester bromide;
naproxen 5-(N,N-di-isopropyl-N-methylammonium)thio pentyl ester bromide;
naproxen 5-(N,N-di-n-butyl-N-methylammonium)thiopentyl ester bromide;
naproxen 5-(N,N-dimethyl-N-benzylammonium)thiopentyl ester bromide;
naproxen 5-(N-phenyl-N-benzyl-N-methylammonium)thiopentyl ester bromide;
naproxen 5-(N-methyl-N-ethyl-N-butylammonium)thiopentyl ester bromide;
naproxen 5-(N-methyl-N-ethyl-N-phenylammonium)thiopentyl ester bromide;
naproxen 6-(4-morpholinyl-N-methylammonium)thiohexyl ester bromide;
naproxen 6-(1-piperidinyl-N-methylammonium)thiohexyl ester bromide;
naproxen 6-(1-pyrrolidinyl-N-methylammonium)thiohexyl ester bromide;
naproxen 6-(N,N-diphenyl-N-methylammonium)thiohexyl ester bromide;

naproxen 6-(N,N-diethyl-N-methylammonium)thiohexyl ester bromide;
naproxen 6-(N,N-di-isopropyl-N-methylammonium)thiohexyl ester bromide;
naproxen 6-(N,N-di-n-butyl-N-methylammonium)thiohexyl ester bromide;
naproxen 6-(N,N-dimethyl-N-benzylammonium)thiohexyl ester bromide;
naproxen 6-(N-phenyl-N-benzyl-N-methylammonium)thiohexyl ester bromide;
naproxen 6-(N-methyl-N-ethyl-N-butylammonium)thiohexyl ester bromide; and
naproxen 6-(N-methyl-N-ethyl-N-phenylammonium)thiohexyl ester bromide.

B.4. Similarly, by substituting: optionally other ω-dialkylamino alkylthiols (such as those listed, for example, in Preparations 2 and 4) for 2-dimethylaminoethane thiol; other ANSAIDs (such as those listed, for example, in Example 2 A.1 and A.2) for naproxen; and optionally other alkylating agents $R^3X$ (such as ethyl iodide, methyl bromide, methyl chloride, and the like) for methyl iodide, the following compounds of Formula I are prepared in accordance with the procedure described in this Example 2, section A.0, above:

(i) From the ω-dialkylamino alkylthiols of formula (3) listed in Preparation 2:
ketorolac 3-(N,N,N-triethylammonium)thioethyl ester chloride;
ketorolac 2-(1-piperidinyl-N-methylammonium)thioethyl ester chloride;
ketorolac 2-(1-pyrrolidinyl-N-methylammonium)thioethyl ester chloride;
ketorolac 2-(4-morpholinyl-N-methylammonium)thioethyl ester chloride;
ketorolac 2-(N,N-dibutyl-N-methylammonium)thioethyl ester chloride;
ketorolac 2-(N,N-dibenzyl-N-methylammonium)thioethyl ester chloride;
indomethacin 2-(N,N-diphenyl-N-methylammonium)thioethyl ester chloride;
indomethacin 2-(N,N-diethyl-N-methylammonium)thioethyl ester chloride;
indomethacin 2-(N,N-di-n-propyl-N-methylammonium)thioethyl ester chloride;
indomethacin 2-(N,N-di-n-butyl-N-methylammonium)thioethyl ester chloride;
indomethacin 2-(N,N-dimethyl-N-benzylammonium)thioethyl ester chloride;
indomethacin 2-(N-phenyl-N-benzyl-N-methylammonium)thioethyl ester chloride;
sulindac 2-(N-methyl-N-ethyl-N-butylammonium)thioethyl ester chloride;
sulindac 2-(N-methyl-N-ethyl-N-phenylammonium)thioethyl ester chloride
sulindac 2-(N,N,N-trimethylammonium)thioethyl ester bromide;
sulindac 2-(1-piperidinyl-N-methylammonium)thioethyl ester bromide;
sulindac 2-(1-pyrrolidinyl-N-methylammonium)thioethyl ester bromide;
sulindac 2-(4-morpholinyl-N-methylammonium)thioethyl ester bromide;
ibuprofen 2-(N,N-dibutyl-N-methylammonium)thioethyl ester bromide;
naproxen 2-(N,N-dibenzyl-N-methylammonium)thioethyl ester bromide;
ibuprofen 2-(N,N-diphenyl-N-methylammonium)thioethyl ester bromide;
ibuprofen 2-(N,N-diethyl-N-methylammonium)thioethyl ester bromide;
ibuprofen 2-(N,N-di-n-propyl-N-methylammonium)thioethyl ester bromide;
ibuprofen 2-(N,N-di-n-butyl-N-methylammonium)thioethyl ester bromide;
zomepirac 2-(N,N-dimethyl-N-benzylammonium)thioethyl ester bromide;
zomepirac 2-(N-phenyl-N-benzyl-N-methylammonium)thioethyl ester bromide;
zomepirac 2-(N-methyl-N-ethyl-N-butylammonium)thioethyl ester bromide; and
zomepirac 2-(N-methyl-N-ethyl-N-phenylammonium)thioethyl ester bromide.

(ii) From the ω-dialkylamino alkylthiols of formula (3) listed in Preparation 4:
zomepirac 3-(N,N-trimethylammonium)thiopropyl ester chloride;
zomepirac 4-(N,N,N-trimethylammonium)thiobutyl ester chloride;
flufenamic acid 6-(N,N,N-trimethylammonium)thiohexyl ester chloride;
anirolac 4-(4-morpholinyl-N-methylammonium)thiobutyl ester chloride;
ketorolac 4-(1-piperidinyl-N-methylammonium)thiobutyl ester chloride;
tifurac 4-(1-pyrrolidinyl-N-methylammonium)thiobutyl ester chloride;
flufenamic acid 4-(N,N-diphenyl-N-methylammonium)thiobutyl ester chloride;
flufenamic acid 4-(N,N-diethyl-N-methylammonium)thiobutyl ester chloride;
flufenamic acid 4-(N,N-di-isopropyl-N-methylammonium)thiobutyl ester chloride;
flufenamic acid 4-(N,N-di-n-butyl-N-methylammonium)thiobutyl ester chloride;
flufenamic acid 4-(N,N-dimethyl-N-benzylammonium)thiobutyl ester chloride;
ketoprofen 4-(N-phenyl-N-benzyl-N-methylammonium)thiobutyl ester chloride;
ketoprofen 4-(N-methyl-N-ethyl-N-butylammonium)thiobutyl ester chloride;
ketoprofen 4-(N-methyl-N-ethyl-N-phenylammonium)thiobutyl ester chloride;
anirolac 3-(4-morpholinyl-N-methylammonium)thiopropyl ester chloride;
ketorolac 3-(1-piperidinyl-N-methylammonium)thiopropyl ester chloride;
tifurac 3-(1-pyrrolidinyl-N-methylammonium)thiopropyl ester chloride;
ketoprofen 3-(N,N-diphenyl-N-methylammonium)thiopropyl ester chloride;
ketoprofen 3-(N,N-diethyl-N-methylammonium)thiopropyl ester chloride;
ketoprofen 3-(N,N-di-isopropyl-N-methylammonium)thiopropyl ester chloride;
benoxaprofen 3-(N,N-di-n-butyl-N-methylammonium)thiopropyl ester chloride;
benoxaprofen 3-(N,N-dimethyl-N-benzylammonium)thiopropyl ester chloride;
benoxaprofen 3-(N-phenyl-N-benzyl-N-methylammonium)thiopropyl ester chloride;

benoxaprofen 3-(N-methyl-N-ethyl-N-butylammonium)thiopropyl ester chloride;
benoxaprofen 3-(N-methyl-N-ethyl-N-phenylammonium)thiopropyl ester chloride;
anirolac 5-(4-morpholinyl-N-methylammonium)thiopentyl ester chloride;
ketorolac 5-(1-piperidinyl-N-methylammonium)thiopentyl ester chloride;
tifurac 5-(1-pyrrolidinyl-N-methylammonium)thiopentyl ester chloride;
benoxaprofen 5-(N,N,N-trimethylammonium)thiopentyl ester chloride;
etodolac 5-(N,N-diphenyl-N-methylammonium)thiopentyl ester chloride;
etodolac 5-(N,N-diethyl-N-methylammonium)thiopentyl ester chloride;
etodolac 5-(N,N-di-isopropyl-N-methylammonium)thio pentyl ester chloride;
etodolac 5-(N,N-di-n-butyl-N-methylammonium)thiopentyl ester chloride;
etodolac 5-(N,N-dimethyl-N-benzylammonium)thiopentyl ester chloride;
etodolac 5-(N-phenyl-N-benzyl-N-methylammonium)thiopentyl ester chloride;
tolmetin 5-(N-methyl-N-ethyl-N-butylammonium)thiopentyl ester chloride;
tolmetin 5-(N-methyl-N-ethyl-N-phenylammonium)thiopentyl ester chloride;
anirolac 6-(4-morpholinyl-N-methylammonium)thiohexyl ester chloride;
ketorolac 6-(1-piperidinyl-N-methylammonium)thiohexyl ester chloride;
tifurac 6-(1-pyrrolidinyl-N-methylammonium)thiohexyl ester chloride;
tolmetin 6-(N,N-diphenyl-N-methylammonium)thiohexyl ester chloride;
tolmetin 6-(N,N-diethyl-N-methylammonium).thiohexyl ester chloride;
tolmetin 6-(N,N-di-isopropyl-N-methylammonium)thiohexyl ester chloride;
tolmetin 6-(N,N-di-n-butyl-N-methylammonium)thiohexyl ester chloride;
tifurac 6-(N,N-dimethyl-N-benzylammonium)thiohexyl ester chloride;
tifurac 6-(N-phenyl-N-benzyl-N-methylammonium)thiohexyl ester chloride;
tifurac 6-(N-methyl-N-ethyl-N-butylammonium)thiohexyl ester chloride;
tifurac 6-(N-methyl-N-ethyl-N-phenylammonium)thiohexyl ester chloride;
tifurac 3-(N,N,N-trimethylammonium)thiopropyl ester bromide;
tifurac 4-(N,N,N-trimethylammonium)thiobutyl ester bromide;
diclofenac 6-(N,N,N-trimethylammonium)thiohexyl ester bromide;
anirolac 4-(4-morpholinyl-N-methylammonium)thiobutyl ester bromide;
ketorolac 4-(1-piperidinyl-N-methylammonium)(thiobutyl ester bromide;
tifurac 4-(1-pyrrolidinyl-N-methylammonium)thiobutyl ester bromide;
diclofenac 4-(N,N-diphenyl-N-methylammonium)thiobutyl ester bromide;
diclofenac 4-(N,N-diethyl-N-methylammonium)thiobutyl ester bromide;
diclofenac 4-(N,N-di-isopropyl-N-methylammonium)thiobutyl ester bromide;
diclofenac 4-(N,N-di-n-butyl-N-methylammonium)thiobutyl ester bromide;
diclofenac 4-(N,N-dimethyl-N-benzylammonium)thiobutyl ester bromide;
fenclofenac 4-(N-phenyl-N-benzyl-N-methylammonium)thiobutyl ester bromide;
fenclofenac 4-(N-methyl-N-ethyl-N-butylammonium)thiobutyl ester bromide;
fenclofenac 4-(N-methyl-N-ethyl-N-phenylammonium)thiobutyl ester bromide;
anirolac 3-(4-morpholinyl-N-methylammonium)thiopropyl ester bromide;
ketorolac 3-(1-piperidinyl-N-methylammonium)thiopropyl ester bromide;
tifurac 3-(1-pyrrolidinyl-N-methylammonium)thiopropyl ester bromide;
fenclofenac 3-(N,N-diphenyl-N-methylammonium)thiopropyl ester bromide;
fenclofenac 3-(N,N-diethyl-N-methylammonium)thiopropyl ester bromide;
fenclofenac 3-(N,N-di-isopropyl-N-methylammonium)thiopropyl ester chloride;
anirolac 3-(N,N-di-n-butyl-N-methylammonium)thiopropyl ester bromide;
anirolac 3-(N,N-dimethyl-N-benzylammonium)thiopropyl ester bromide;
anirolac 3-(N-phenyl-N-benzyl-N-methylammonium)thiopropyl ester bromide;
anirolac 3-(N-methyl-N-ethyl-N-butylammonium)thiopropyl ester bromide;
anirolac 3-(N-methyl-N-ethyl-N-phenylammonium)thiopropyl ester bromide;
anirolac 5-(4-morpholinyl-N-methylammonium)thiopentyl ester bromide;
ketorolac 5-(1-piperidinyl-N-methylammonium)thiopentyl ester bromide;
tifurac 5-(1-pyrrolidinyl-N-methylammonium)thiopentyl ester bromide;
anirolac 5-(N,N,N-trimethylammonium)thiopentyl ester bromide;
mefenamic acid 5-(N,N-diphenyl-N-methylammonium)thiopentyl ester bromide;
mefenamic acid 5-(N,N-diethyl-N-methylammonium)thiopentyl ester bromide;
mefenamic acid 5-(N,N-di-isopropyl-N-methylammonium)thiopentyl ester bromide;
mefenamic acid 5-(N,N-di-n-butyl-N-methylammonium)thiopentyl ester bromide;
mefenamic acid 5-(N,N-dimethyl-N-benzylammonium)thiopentyl ester bromide;
mefenamic acid 5-(N-phenyl-N-benzyl-N-methylammonium)thiopentyl ester bromide;
ketorolac 5-(N-methyl-N-ethyl-N-butylammonium)thiopentyl ester bromide;
ketorolac 5-(N-methyl-N-ethyl-N-phenylammonium)thiopentyl ester bromide;
anirolac 6-(4-morpholinyl-N-methylammonium)thiohexyl ester bromide;
ketorolac 6-(1-piperidinyl-N-methylammonium)thiohexyl ester bromide;
tifurac 6-(1-pyrrolidinyl-N-methylammonium)thiohexyl ester bromide;

ketorolac 6-(N,N-diphenyl-N-methylammonium)thiohexyl ester bromide;
ketorolac 6-(N,N-diethyl-N-methylammonium)thiohexyl ester bromide;
anirolac 6-(N,N-di-isopropyl-N-methylammonium)thiohexyl ester bromide;
anirolac 6-(N,N-di-n-butyl-N-methylammonium)thiohexyl ester bromide;
anirolac 6-(N,N-dimethyl-N-benzylammonium)thiohexyl ester bromide;
anirolac 6-(N-phenyl-N-benzyl-N-methylammonium)thiohexyl ester bromide;
tifurac 6-(N-methyl-N-ethyl-N-butylammonium)thiohexyl ester bromide; and
tifurac 6-(N-methyl-N-ethyl-N-phenylammonium)thiohexyl ester bromide.

It will be apparent to one of ordinary skill in the art that the above listing is for the purpose of exemplification, and is not exhaustive. Accordingly, any of the ANSAIDs listed in parts A.1 and 2 of this Example 2 can be used in place of naproxen to obtain the analogous esters to the naproxen esters listed in parts B.2 and B.3 of this Example 2. Moreover, other ANSAIDS (RCOOH) can be substituted, as well as other dialkylaminoalkyl thiols of formula (3), as well as other alkylating agents $R^3X$.

B.5. By treatment of the intermediate oily ester with methyl chloride in a pressure apparatus at 60°C for 48 hours, the following compound was prepared:

naproxen thiocholine ester chloride, mp 110-112°C.

EXAMPLE 3

NAPROXEN CHOLINE ESTER BROMIDE BY REACTION SCHEME E FROM THE INTERMEDIATE OF PREPARATION 5

Naproxen 2-bromoethyl ester (6.7 g, 20 mmol) and condensed trimethylamine (25 ml) were combined in a sealed pressure tube (Ace glass, threaded top) and heated to 50°C overnight. The tube was cooled in ice, opened, and the residue was triturated with diethyl ether (100 ml) to afford the title compound upon filtration and drying.

By use of the appropriate tertiary amine $R^1R^2R^3N$ in place of trimethylamine, other compounds of Formula I where M = O may be prepared.

By use of the appropriate tertiary amine and the appropriate ANSAID ω-haloalkylester, other compounds of Formula I where M = O may be prepared.

EXAMPLE 4

NAPROXEN CHOLINE ESTER CHLORIDE BY REACTION SCHEME F

Naproxen acid chloride prepared from naproxen (11.5 g) and oxalyl chloride (6.5 ml) in benzene (200 ml) in the presence of a catalytic amount of DMF (0.5 ml) followed by evaporation, was dissolved in dry dimethyformamide (100 ml), and was treated with choline chloride (7.7 g) and triethylamine (8.5 ml). After warming to 50°C for 2 hours, the mixture was cooled, evaporated, and recrystallized from hot acetone to yield the title compound.

Substitution of thiocholine chloride affords naproxen thiocholine ester chloride.

Substitution of acid chlorides derived from other ANSAIDs affords the corresponding choline or thiocholine ester chlorides.

In the following Examples 5 - 13, the active ingredient is a compound of Formula I, preferably selected from the more preferred or most preferred classes of the compounds of Formula I, and most preferably naproxen thiocholine ester chloride.

EXAMPLE 5

An injectable preparation buffered to a pH of 7 is prepared having the following composition:

| Ingredients | |
|---|---|
| Active ingredient | 0.2 g |
| $KH_2PO_4$ buffer (0.4 M solution) | 2 ml |
| KOH (1 N) | q.s. to pH 7 |
| water (distilled, sterile) | q.s. to 20 ml |

EXAMPLE 6

An oral suspension is prepared having the following composition:

| Ingredients | |
|---|---|
| Active ingredient | 5.0 g |
| fumaric acid | 0.5 g |
| sodium chloride | 2.0 g |
| methyl paraben | 0.1 g |
| granulated sugar | 25.5 g |
| sorbitol (70% solution) | 12.85 g |
| Veegum K (Vanderbilt Co.) | 1.0 g |
| flavoring | 0.035 ml |

colorings 0.5 mg
distilled water q.s. to 100 ml

EXAMPLE 7

| Ingredients | Quantity per tablet, mgs. |
|---|---|
| Active ingredient | 25 |
| cornstarch | 20 |
| lactose, spray-dried | 153 |
| magnesium stearate | 2 |

The above ingredients are throughly mixed and pressed into single scored tablets.

EXAMPLE 8

| Ingredients | Quantity per tablet, mgs. |
|---|---|
| Active ingredient | 100 |
| lactose, spray-dried | 148 |
| magnesium stearate | 2 |

The above ingredients are mixed and introduced into a hard-shell gelatin capsule.

EXAMPLE 9

| Ingredients | Quantity per tablet, mgs. |
| --- | --- |
| Active ingredient | 200 |
| cornstarch | 50 |
| lactose | 145 |
| magnesium stearate | 5 |

The above ingredients are mixed intimately and pressed into a single scored tablets.

EXAMPLE 10

| Ingredients | Quantity per tablet, mgs. |
|---|---|
| Active ingredient | 108 |
| lactose | 15 |
| cornstarch | 25 |
| magnesium stearate | 2 |

The above ingredients are mixed and introduced into a hard-shell gelatin capsule.

EXAMPLE 11

| Ingredients | Quantity per tablet, mgs. |
|---|---|
| Active ingredient | 150 |
| lactose | 92 |

The above ingredients are mixed and introduced into a hard-shell gelatin capsule.

EXAMPLE 12

Topical Formulation

| Ingredients | | grams |
|---|---|---|
| Active ingredient | | 0.2-2 |
| Span 60 | | 2 |
| Tween 60 | | 2 |
| Mineral oil | | 5 |
| Petrolatum | | 10 |
| Methyl paraben | | 0.15 |
| Propyl paraben | | 0.05 |
| BHA (butylated hydroxy anisole) | | 0.01 |
| Water | q.s. | 100 |

All of the above ingredients, except water, are combined and heated to 60° C with stirring. A sufficient quantity of water at 60° C is then added with vigorous stirring to emulsify the ingredients, and water then added q.s. 100 g.

EXAMPLE 13

Topical Formulation

| Ingredients | wt-% |
|---|---|
| Active ingredient | 2.5 |
| Klucel (hydroxypropylcellulose) | 2.5 |
| diisopropyl adipate | 10 |
| ethanol | 80 |
| propylene glycol | 5 |

All of the ingredients except Klucel are first mixed together at room temperature, so that the active ingredient dissolves. Then the Klucel is dispersed and left to gel overnight.

EXAMPLE 14

Test for Ability to Cause Gastro-Intestinal Erosion In Rats

Male Lai: COX (SD) rats weighing 200-250 gm were acclimated for one week under standard laboratory conditions. Each animal was individually indentified and body weights were determined on the first day of the test and again at autopsy. The test materials, suspended in aqueous carboxymethyl cellulose vehicle, were administered orally once daily in a volume of 1.0ml per 100g body weight for four consecutive days. Food was withdrawn from the animals following the last dose and the animals were sacrificed 24 hours later. At necropsy, the stomach and intestines were removed and observed for lesions. A group of five rats was used for each dose level of test material, and erosions were graded on a 0-10 scale with a score of 10 indicating that the animal died of ulceration prior to autopsy. The incidence of lesions for each dose level tested is reported.

Induction of Erosions and Ulcerations of the Intestinal Tract of Rats

| Test Material | Daily Dose mg/kg | Mean Intestinal Ulcer Score ± 1Std. Dev. |
|---|---|---|
| Naproxen | 20 | 1.6 ± 0.9 |
| | 25 | 3.2 ± 1.1 |
| | 30 | 6.4 ± 1.7 |
| | 35 | 7.6 ± 0.9 |
| Naproxen thiocholine ester iodide | 150 | 0 |
| | 200 | 0.4 ± 0.9 |
| | 250 | 1.6 ± 1.7 |
| | 300 | 3.0 ± 1.7 |
| | 400 | 7.2 ± 1.1 |
| Naproxen thiocholine ester chloride | 200 | 0.4 ± 0.9 |
| | 300 | 1.6 ± 0.9 |
| | 400 | 5.6 ± 2.2 |
| Indomethacin | 5 | 7.2 ± 1.9 |
| Indomethacin thiocholine ester iodide | 25 | 0 |
| | 100 | 0 |
| Ketorolac | 5 | 1.9 ± 0.9 |
| | 10 | 4.8 ± 1.7 |
| | 20 | 7.4 ± 1.7 |
| Ketorolac thiocholine ester iodide | 25 | 2.0 ± 2.0 |
| | 50 | 6.4 ± 0.9 |
| | 75 | 7.2 ± 1.1 |

EXAMPLE 15

Test for Activity In the Rat Adjuvant-Induced Arthritis Assay

Female Crl:CD, br rats weighting 160-180 gm were randomly distributed to treatment groups of 12 animals, and were given food and water ad libitum. Test materials were prepared fresh weekly as suspensions in aqueous carboxymethyl cellulose. Animals were orally dosed with volumes of 1 ml twice per day Monday through Friday, and with 2 ml once per day on Saturdays and Sundays. At time 0, rats were injected intradermally in the proximal quarter of the tail with 0.1 ml of a mineral oil suspension of heat killed and dried Mycobacterium butyricum (Difco) at a concentration of 10 mg/ml. After sacrifice, the hind paws of each animal were removed and weighed.

Inhibition of Adjuvant-Induced Arthritis in the Rat

| Test Material | Daily Dose mg/kg | Percent Inhibition of Hind Paw Weight |
|---|---|---|
| Naproxen | 1.4 | 22 |
| | 3.5 | 70 |
| | 7.0 | 77 |
| Naproxen thiocholine ester iodide | 7.0 | 40 |
| | 10 | 69 |
| | 14 | 40,53* |
| | 28 | 51,75* |
| Ketorolac | 0.1 | 5 |
| | 0.5 | 14 |
| | 2.5 | 59 |
| Ketorolac thiocholine ester iodide | 1.0 | 10 |
| | 5.0 | 79,56* |
| Indomethacin | 2.0 | 75 |
| Indomethacin thiocholine ester iodide | 7.0 | 15 |
| | 14 | 59 |

*Results of two independent tests.

EXAMPLE 16

Protective Effect of Orally Administered Naproxen Thiocholine Ester Iodide Against Intestinal Ulceration in Rats Induced by the Subcutaneous Administration of Indomethacin

Male Crl:D,br rats weighing approximately 200 gm received indomethacin, 4 mg/kg, injected subcutaneously each day for four consecutive days to induce the formation of erosions and ulcers of the small intestine. Food was removed following the last dose of indomethacin and the rats were necropsied 18-24 hours later. Erosions of small intestine were scored as follows:

0 - no detectable lesions
2 - small ulcerattions (1-15) less than 1 mm
4 - small ulcerations plus 1-10 medium (2-3 mm) ulcerations
6 - predominantly medium ulceratiaons plus 2-5 severe ($>$ 4mm) ulcers without evidence of perforation
8 - many ($>$ 20) medium and severe ulcerations with evidence of perforation
10 - animals dying during assay as a consequence of intestinal perforation and septicemia.

Agents to be tested for protective effects were administered orally in an aqueous vehicle, with one-half the daily dose being administered 4 hours prior to indomethacin injection and one half the daily dose being administered at the time of indomethacin injection.

Protective Effect of Orally Administered Naproxen Thiocholine Ester Iodide Against Intestinal Ulceration in Rats Induced by the Subcutaneous Administration of Indomethacin (4 mg/kg/day)

| Test Material | Oral Dose mg/kg/day | No. of Rats | Intestinal Erosion Score Mean ± S.D. |
|---|---|---|---|
| Vehicle | - | 10 | 5.8 ± 1.8 |
| Naproxen Thiocholine Ester Iodide | 5 | 5 | 4.0 ± 2.4 |
| | 10 | 5 | 2.4 ± 0.9 |
| | 20 | 10 | 1.8 ± 2.4 |
| | 50 | 5 | 0.8 ± 1.1 |

EXAMPLE 17

Five male Hsd:(SD)BR rats weighing 205-215 g were acclimated for approximately one week prior to use under standard laboratory conditions. Naproxen thiocholine ester iodide, suspended in aqueous carboxymethylcellulose vehicle was administered orally twice daily in a volume of 1.0 ml per 200 g body weight for 21 consecutive days at a total daily dose of 100 mg/Kg. The compound did not produce any gross signs of toxicity. The other compounds of the present invention also do not exhibit any toxic effects.

**Claims**

1. A compound of the formula:

$RC(O)M(CH_2)_nN^+R^1R^2R^3X^-$ (I)

wherein:

R is a radical such that RCOOH is an acidic nonsteroidal antiinflammatory drug (ANSAID):

M is O or S;

n is an integer from 2 to 6;

$R^1$ and $R^2$ are independently alkyl of 1 to 6 carbon atoms having a $-CH_2-$ group directly connected to the nitrogen atom, phenyl, or benzyl;

or $R^1$ and $R^2$ may be combined with the nitrogen atom to which they are attached to form morpholino or a 5- or 6- membered satured heterocyclic ring;

$R^3$ is alkyl of 1 to 6 carbon atoms having a $-CH_2-$ group directly connected to the nitrogen atom, or benzyl;

and X is a pharmaceutically acceptable anion.

2. A compound f Claim 1 wherein $R^1$, $R^2$ and $R^3$ are independently alkyl of 1 to 6 carbon atoms, preferably alkyl of 1 to 4 carbon atoms, most preferably methyl.

3. A compound of Claim 1 or Claim 2 wherein X is halo preferably chloro.

4. A compound of any one of the preceding claims wherein n is 2 or 3.

5. A compound of Claim 4 wherein n is 2, $R^1$, $R^2$ and $R^3$ are methyl, and X is halo preferably chloro.

6. A compound of any one of the preceding claims wherein RC(O)- is a radical such that RCOOH is an

acidic nonsteroidal antiinflammatory agent selected from the group consisting of indomethacin, sulindac, tolmetin, tifurac, zomepirac, diclofenac, fenclofenac, etodolac, ibuprofen, naproxen, ketoprofen, benoxaprofen, mefenamic acid, flufenamic acid, ketorolac and anirolac, preferably an acidic nonsteroidal antiinflammatory agent selected from the group consisting of naproxen, ketorolac, anirolac, tifurac, diclofenac, ibuprofen, indomethacin, and sulindac and most preferably an acidic nonsteroidal antiinflammatory agent selected from the group consisting of naproxen, ketorolac, anirolac, tifurac and indomethacin.

7. A compound of Claim 6 wherein RC(O) is a radical such that RCOOH is naproxen.

8. A compound of Claim 7 wherein $R^1$, $R^2$ and $R^3$ are methyl, n is 2 or 3, and X is halo.

9. A compound of Claim 8 wherein n is 2, X is Cl, I or Br and M is S, namely, naproxen thiocholine ester chloride, naproxen thiocholine ester iodide and naproxen thiocholine ester bromide respectively.

10. A compound of Claim 8 wherein n is 2, X is Cl and M is O, namely, naproxen choline ester chloride.

11. A compound of Claim 6 wherein RC(O) is a radical such that RCOOH is ketorolac.

12. A compound of Claim 6 wherein RC(O) is a radical such that RCOOH is anirolac.

13. A compound of Claim 6 wherein RC(O) is a radical such that RCOOH is tifurac.

14. A compound of Claim 6 wherein RC(O) is a radical such that RCOOH is indomethacin.

15. A compound of Claim 6 wherein RC(O) is a radical such that RCOOH is ibuprofen.

16. A compound of Claim 6 wherein RC(O) is a radical such that RCOOH is diclofenac.

17. A compound of Claim 6 wherein RC(O) is a radical such that RCOOH is sulindac.

18. A compound of any one of claims 11 to 17 wherein $R^1$, $R^2$ and $R^3$ are methyl, n is 2 or 3 and X is halo.

19. A compound according to claim 18 wherein n is 2 and X is chloro.

20. A compound of Claim 6 wherein RC(O) is a radical such that RCOOH is etodolac, $R^1$, $R^2$ and $R^3$ are methyl, n is 2, X is I, and M is S or O, namely, etodolac thiocholine ester iodide and etodolac choline ester iodide respectively.

21. A compound of Claim 1 wherein RC(O) is a radical such that RCOOH is naproxen, $R^1$, $R^2$ and $R^3$ are methyl, n is 5, M is O an X is I, namely, naproxen 5-(N,N,N-trimethylammonium)n-pentyl ester iodide.

22. A compound of Claim 1 wherein RC(O) is a radical such that RCOOH is ketorolac, $R^1$, $R^2$ and $R^3$ are methyl, n is 4, M is O and X is I, namely ketorolac 4-(N,N,N-trimethylammonium)n-butyl ester iodide.

23. A compound of Claim 1 selected from:
naproxen choline ester chloride;
ketorolac choline ester chloride;
anirolac choline ester chloride;
diclofenac choline ester chloride;
ibuprofen choline ester chloride;
indomethacin choline ester chloride;
sulindac choline ester chloride;
tifurac choline ester chloride;
naproxen thiocholine ester chloride;
ketorolac thiocholine ester chloride;
anirolac thiocholine ester chloride;
diclofenac thiocholine ester chloride;
ibuprofen thiocholine ester chloride;
indomethacin thiocholine ester chloride;
sulindac thiocholine ester chloride;
tifurac thiocholine ester chloride;
naproxen choline ester bromide;
ketorolac choline ester bromide;
anirolac choline ester bromide;
diclofenac choline ester bromide;
ibuprofen choline ester bromide;
indomethacin choline ester bromide;
sulindac choline ester bromide;
tifurac choline ester bromide;
naproxen thiocholine ester bromide;
ketorolac thiocholine ester bromide;
anirolac thiocholine ester bromide;
diclofenac thiocholine ester bromide;
ibuprofen thiocholine ester bromide;
indomethacin thiocholine ester bromide;
sulindac thiocholine ester bromide;
tifurac thiocholine ester bromide;
naproxen choline ester iodide;
ketorolac choline ester iodide;
anirolac choline ester iodide;
diclofenac choline ester iodide;

ibuprofen choline ester iodide;
indomethacin choline ester iodide;
sulindac choline ester iodide;
tifurac choline ester iodide;
naproxen thiocholine ester iodide;
ketorolac thiocholine ester iodide;
anirolac thiocholine ester iodide;
diclofenac thiocholine ester iodide;
ibuprofen thiocholine ester iodide;
indomethacin thiocholine ester iodide;
sulindac thiocholine ester iodide;
tifurac choline ester iodide; and
naproxen 5-(N,N,N-trimethylammonium)pentyl ester iodide
ketorolac 4-(N,N,N-trimethylammonium)butyl ester iodide;
etodolac choline ester iodide; and
etodolac thiocholine ester iodide.

24. A compound of any one of the preceding claims for the treatment of inflammation in a mammal.

25. A pharmaceutical composition comprising a therapeutically effective amount of a compound of any one of the preceding claims in admixture with a pharamceutically acceptable excipient.

26. A pharmaceutical composition comprising a therapeutically effective combined amount of a compound of any one of claims 1 to 24 in admixture with a nonsteroidal antiinflammatory drug and a pharmaceutically acceptable excipient.

27. A pharmaceutical preparation comprising a compound of any one of claims 1 to 24 and a non-steroidal anti-inflammatory drug (NSAID), said compound being arranged for simultaneous or sequential administration with NSAID.

28. A pharmaceutical composition according to claim 26, or a pharmaceutical preparation according to claim 27 wherein said NSAID is an acidic non-steroidal antiinflammatory drug, or a pharmaceutically acceptable salt

29. A pharmaceutical composition or preparation according to claim 28 wherein the NSAID and the R group of the compound of formula I are chosen such that the NSAID is RCOOH, or a pharmaceutically acceptable salt thereof.

30. Use of a compound of any one of claims 1 to 24 in the manufacture of a medicament for the treatment of inflammation in a mammal.

31. Use according to claim 30 wherein said inflammation to be treated is a symptom of arthritis.

32. Use according to claim 30 or claim 31 wherein said medicament comprises, in addition to said compound a non-steroidal anti-inflammatory drug.

33. A process for preparing a compound of the formula

$$RC(O)M(CH_2)_nN^+R^1R^2R^3\,X^- \quad (I)$$

wherein:

R is a radical such that RCOOH is an acidic nonsteroidal antiinflammatory drug (ANSAID):

M is O or S;

n is an integer from 2 to 6;

$R^1$ and $R^2$ are independently alkyl of 1 to 6 carbon atoms having a $-CH_2-$ group directly connected to the nitrogen atom, phenyl, or benzyl;

or $R^1$ and $R^2$ may be combined with the nitrogen atom to which they are attached to form morpholino or a 5- or 6- membered saturated heterocyclic ring;

$R^3$ is alkyl of 1 to 6 carbon atoms having a $-CH_2-$ group directly connected to the nitrogen atom, or benzyl;

and X is a pharmaceutically acceptable anion.

which comprises:

a) alkylating a compound of the formula

$$RC(O)M(CH_2)_nNR^1R^2$$

with a compound of the formula $R^3X$ wherein R, M, n, $R^1$, $R^2$, $R^3$ and X are as defined above; or

b) reacting a compound of the formula

$$RC(O)M(CH_2)_nX$$

with a compound of the formula

$$R^1R^2R^3N$$

wherein R, M, n, X, $R^1$, $R^2$ and $R^3$ are as defined above; or

c) reacting a compound of the formula

RC(O)OH

or a derivative thereof with a compound of the formula

$HM(CH_2)_nN^+R^1R^2R^3\ X^-$

wherein R, M, n, $R^1$, $R^2$, $R^3$ and X are as defined above; or

d) reacting a compound of the formula

RC(O)OR′

with a compound of the formula

$H(M)(CH_2)_nN^+R^1R^2R^3\ X^-$

wherein R, M n, $R^1$, $R^2$, $R^3$ and X are as defined above and R′ is a alkyl of 1-4 carbon atoms.

**Claims for the following Contracting States: GR, ES**

1. A process for preparing a compound of the formula

$RC(O)M(CH_2)_nN^+R^1R^2R^3\ X^-$ (I)

wherein:

R is a radical such that RCOOH is an acidic nonsteroidal antiinflammatory drug (ANSAID):

M is O or S;

n is an integer from 2 to 6;

$R^1$ and $R^2$ are independently alkyl of 1 to 6 carbon atoms having a $-CH_2-$ group directly connected to the nitrogen atom, phenyl, or benzyl;

or $R^1$ and $R^2$ may be combined with the nitrogen atom to which they are attached to form morpholino or a 5- or 6- membered saturated heterocyclic ring;

$R^3$ is alkyl of 1 to 6 carbon atoms having a $-CH_2-$ group directly connected to the nitrogen atom, or benzyl;

and X is a pharmaceutically acceptable anion.

which comprises:

a) alkylating a compound of the formula

$RC(O)M(CH_2)_nNR^1R^2$

with a compound of the formula $R^3X$ wherein R, M, n, $R^1$, $R^2$, $R^3$ and X are as defined above; or

b) reacting a compound of the formula

$RC(O)M(CH_2)_nX$

with a compound of the formula

$R^1R^2R^3N$

wherein R, M, n, X, $R^1$, $R^2$ and $R^3$ are as defined above; or

c) reacting a compound of the formula

RC(O)OH

or a derivative thereof with a compound of the formula

$HM(CH_2)_nN^+R^1R^2R^3\ X^-$

wherein R, M, n, $R^1$, $R^2$, $R^3$ and X are as defined above; or

d) reacting a compound of the formula

RC(O)OR′

with a compound of the formula

$H(M)(CH_2)_nN^+R^1R^2R^3\,X^-$

wherein R, M n, $R^1$, $R^2$, $R^3$ and X are as defined above and R′ is an alkyl of 1-4 carbon atoms.

2. A process according to Claim 1 wherein $R^1$, $R^2$ and $R^3$ are independently alkyl of 1 to 6 carbon atoms, preferably alkyl of 1 to 4 carbon atoms, most preferably methyl.

3. A process according to Claim 1 or Claim 2 wherein X is halo preferably chloro.

4. A process according to any one of the preceding claims wherein n is 2 or 3.

5. A process according to Claim 4 wherein n is 2, $R^1$, $R^2$ and $R^3$ are methyl, and X is halo preferably chloro.

6. A process according to any one of the preceding claims wherein RC(O)- is a radical such that RCOOH is an acidic nonsteroidal antiinflammatory agent selected from the group consisting of indomethacin, sulindac, tolmetin, tifurac, zomepirac, diclofenac, fenclofenac, etodolac, ibuprofen, naproxen, ketoprofen, benoxaprofen, mefenamic acid, flufenamic acid, ketorolac and anirolac, preferably an acidic nonsteroidal antiinflammatory agent selected from the group consisting of naproxen, ketorolac, anirolac, tifurac, diclofenac, ibuprofen, indomethacin, and sulindac and most preferably an acidic nonsteroidal antiinflammatory agent selected from the group consisting of naproxen, ketorolac, anirolac, tifurac and indomethacin.

7. A process according to Claim 6 wherein RC(O) is a radical such that RCOOH is naproxen.

8. A process according to Claim 7 wherein $R^1$, $R^2$ and $R^3$ are methyl, n is 2 or 3, and X is halo.

9. A process according to Claim 8 wherein n is 2, X is Cl, I or Br and M is S, namely, naproxen thiocholine ester chloride, naproxen thiocholine ester iodide and naproxen thiocholine ester bromide respectively.

10. A process according to Claim 8 wherein n is 2, X is Cl and M is O, namely, naproxen choline ester chloride.

11. A process according to Claim 6 wherein RC(O) is a radical such that RCOOH is keterolac.

12. A process according to Claim 6 wherein RC(O) is a radical such tat RCOOH is anirolac.

13. A process according to Claim 6 wherein RC(O) is a radical such that RCOOH is tifurac.

14. A process according to of Claim 6 wherein RC(O) is a radical such that RCOOH is indomethacin.

15. A process according to Claim 6 wherein RC(O) is a radical such that RCOOH is ibuprofen.

16. A process according to Claim 6 wherein RC(O) is a radical such that RCOOH is diclofenac.

17. A process according to Claim 6 wherein RC(O) is a radical such that RCOOH is sulindac.

18. A process according to and one of claims 11 to 17 wherein $R^1$, $R^2$ and $R^3$ are methyl, n is 2 or 3 and X is halo.

19. A process according to claim 18 wherein n is 2 and X is chloro.

20. A process according to Claim 6 wherein RC(O) is a radical such that RCOOH is etodolac, $R^1$, $R^2$ and $R^3$ are methyl, n is 2, X is I, and M is S or O, namely, etodolac thiocholine ester iodide and etodolac choline ester iodide respectively.

21. A process according to Claim 1 wherein RC(O) is a radical such that RCOOH is naproxen, $R^1$, $R^2$ and $R^3$ are methyl, n is 5, M is O and X is I, namely, naproxen 5-(N,N,N-trimethylammonium)n-pentyl ester iodide.

22. A process according to Claim 1 wherein RC(O) is a radical such that RCOOH is ketorolac, $R^1$, $R^2$ and $R^3$ are methyl, n is 4, M is O and X is I, namely, ketorolac 4-(N,N,N-trimethylammonium)n-butyl ester iodide.

23. A process according to Claim 1 wherein said compound of formula I is selected from
naproxen choline ester chloride;
ketorolac choline ester chloride;
anirolac choline ester chloride;
diclofenac choline ester chloride;
ibuprofen choline ester chloride;
indomethacin choline ester chloride;
sulindac choline ester chloride;
tifurac choline ester chloride;
naproxen thiocholine ester chloride;
ketorolac thiocholine ester chloride;
anirolac thiocholine ester chloride;
diclofenac thiocholine ester chloride;
ibuprofen thiocholine ester chloride;
indomethacin thiocholine ester chloride;
sulindac thiocholine ester chloride;
tifurac thiocholine ester chloride;
naproxen choline ester bromide;
ketorolac choline ester bromide;
anirolac choline ester bromide;
diclofenac choline ester bromide;
ibuprofen choline ester bromide;

indomethacin choline ester bromide;
sulindac choline ester bromide;
tifurac choline ester bromide;
naproxen thiocholine ester bromide;
ketorolac thiocholine ester bromide;
anirolac thiocholine ester bromide;
diclofenac thiocholine ester bromide;
ibuprofen thiocholine ester bromide;
indomethacin thiocholine ester bromide;
sulindac thiocholine ester bromide;
tifurac thiocholine ester bromide;
naproxen choline ester iodide;
ketorolac choline ester iodide;
anirolac choline ester iodide;
diclofenac choline ester iodide;
ibuprofen choline ester iodide;
indomethacin choline ester iodide;
sulindac choline ester iodide;
tifurac choline ester iodide;
naproxen thiocholine ester iodide;
ketorolac thiocholine ester iodide;
anirolac thiocholine ester iodide;
diclofenac thiocholine ester iodide;
ibuprofen thiocholine ester iodide;
indomethacin thiocholine ester iodide;
sulindac thiocholine ester iodide;
tifurac thiocholine ester iodide; and
naproxen 5-(N,N,N-trimethylammonium)pentyl ester iodide;
ketorolac 4-(N,N,N-trimethylammonium)butyl ester iodide;
etodolac choline ester iodide; and
etodolac thiocholine ester iodide.

24. A process comprising production of a compound of formula

$RC(O)M(CH_2)_nN^+R^1R^2R^3\,X^-$ (I)

wherein:

R is a radical such that RCOOH is an acidic nonsteroidal antiinflammatory drug (ANSAID):

M is O or S;

n is an integer from 2 to 6;

$R^1$ and $R^2$ are independently alkyl of 1 to 6 carbon atoms having a $-CH_2-$ group directly connected to the nitrogen atom, phenyl, or benzyl;

or $R^1$ and $R^2$ may be combined with the nitrogen atom to which they are attached to form morpholino or a 5- or 6- membered saturated heterocyclic ring;

$R^3$ is alkyl of 1 to 6 carbon atoms having a $-CH_2-$ group directly connected to the nitrogen atom, or benzyl;

and X is a pharmaceutically acceptable anion.

25. A method of producing a pharmaceutical composition, the method comprising mixing a therapeutically effective amount of a compound produced by the process of any one of the preceding claims with a pharmaceutically acceptable excipient.

26. A method of producing a pharmaceutical composition, the method comprising mixing a therapeutically effective combined amount of a compound produced by the process of any one of claims 1 to 24 with a nonsteroidal antiinflammatory drug and a pharmaceutically acceptable excipient.

27. A method of producing a pharmaceutical preparation the method comprising arranging a compound produced by the process of any one of claims 1 to 24 for simultaneous or sequential administration with a non-steroidal anti-inflammatory drug (NSAID).

28. A method according to claim 26 or claim 27 wherein said NSAID is an acidic non-steroidal anti-inflammatory drug or a pharmaceutically acceptable salt thereof.

29. A method according to claim 28 wherein the NSAID and the R group of the compound of formula I are chosen such that the NSAID is RCOOH, or a pharmaceutically acceptable salt thereof.

30. Use of a compound produced by the process of any one of claims 1 to 24 in the manufacture of a medicament for the treatment of inflammation in a mammal.

31. Use according to claim 30 wherein said inflammation to be treated is a symptom of arthritis.

32. Use according to claim 30 or claim 31 wherein said medicament comprises, in addition to said compound a non-steroidal anti-inflammatory drug.